# EUROPEAN PATENT APPLICATION

(11) **EP 0 948 946 A1**
(43) Date of publication of application: **13.10.1999**
(21) Application number: 99113203.6
(22) Date of filing: 10.08.1995
(51) Int. Cl.: A61F 2/06

(54) **Apparatus and methods for deployment release of intraluminal protheses**

(30) Priority: 12.08.1994 US 290021; 07.06.1995 US 475200
(62) Divisional of application: 95112564.0
(71) Applicant: CARDIOVASCULAR CONCEPTS, INC., Portola Valley, CA 94028 (US)
(72) Inventor: Lenker, Jay A., Los Altos Hills, California 94024 (US); Evans, Michael A., Palo Alto, California 94301 (US); Kim, Steven W., Sunnyvale, California 94086 (US); Glynn, Brian, Sunnyvale, California 94086 (US); Watanabe, Gwendolyn A., Mountain View (US); Freislinger, Kirsten, Palo Alto, California 94301 (US); Ryan, Timothy J., Los Gatos, California 95032 (US); Zarins, Christopher K., Portola Valley, California 94028 (US); Murphy, Richard O., Mountain View, California 94043 (US)
(74) Representative: Sparing - Röhl - Henseler Patentanwälte

(57) **Abstract**

A delivery catheter for a radially compressible tubular prosthesis comprises an elongate shaft slidably received within an elongate sheath. The prosthesis is carried over the distal end of the shaft where it is contained in a radially compressed configuration by the sheath. After introducing the catheter to a desired target location within a body lumen, the prosthesis may be released by proximally retracting the sheath. Optionally, the prosthesis will remain anchored to the shaft during at least part of the release procedure, permitting the user to recapture the prosthesis by distally advancing the sheath. Additionally, an alternative delivery catheter for a radially compressible tubular prosthesis is provided, comprising an elongate shaft slidably received within a tubular cover. The prosthesis is carried within a plurality of elongate, relatively hard runners, and is restrained in a radially compressed configuration by the cover. The prosthesis may be released by proximally retracting the cover. The runners may optionally remain disposed about the prosthesis to be retracted separately, or, alternatively, the runners retract proximally with the cover as the prosthesis slides over the hard runner surfaces.

## Description

### BACKGROUND OF THE INVENTION

### 1. Field of the Invention

The present invention relates generally to apparatus and methods for the endoluminal placement of resilient tubular prostheses, such as grafts, stents, stent-grafts, and other structures. More particularly, the present invention relates to a delivery catheter for the initial placing and optional repositioning of such intraluminal tubular protheses in body lumens, including blood vessels, for the treatment of abdominal and other aneurysms.

Vascular aneurysms are the result of abnormal dilation of a blood vessel, usually resulting from disease and/or genetic predisposition which can weaken the arterial wall and allow it to expand. While aneurysms can occur in any blood vessel, most occur in the aorta and peripheral arteries, with the majority of aortic aneurysms occurring in the abdominal aorta, usually beginning below the renal arteries and often extending distally into one or both of the iliac arteries.

Aortic aneurysms are most commonly treated in open surgical procedures where the diseased vessel segment is bypassed and repaired with an artificial vascular graft. While considered to be an effective surgical technique, particularly considering the alternative of a usually fatal ruptured abdominal aortic aneurysm, conventional vascular graft surgery suffers from a number of disadvantages. The surgical procedure is complex and requires experienced surgeons and well equipped surgical facilities. Even with the best surgeons and equipment, however, patients being treated frequently are elderly and weakened from cardiovascular and other diseases, reducing the number of eligible patients. Even for eligible patients prior to rupture, conventional aneurysm repair has a relatively high mortality rate, usually from 3% to 10%. Morbidity related to the conventional surgery includes myocardial infarction, renal failure, impotence, paralysis, and other conditions. Additionally, even with successful surgery, recovery takes several weeks, and often requires a lengthy hospital stay.

In order to overcome some or all of these drawbacks, endovascular graft placement for the treatment of aneurysms has been proposed. Although very promising, many of the proposed methods and apparatus suffer from other problems. In particular, delivery and placement of the endovascular graft within the vasculature can be problematic. Proper positioning and sizing of the endovascular graft is critical to the successful treatment of an aneurysm. With many endovascular graft structures and their associated delivery catheters, it is difficult or impossible to retract a partially released graft structure. Thus, improper initial placement of a vascular graft can sometimes require open surgical procedures for correction. Additionally, proper sizing of the graft can require maintenance of a large inventory of graft delivery catheters, where each catheter carries a graft having a different length and/or expansible diameter.

Furthermore, grafts are often resilient, biased to expand and anchor the graft within the body lumen. These resiliently expanding grafts are tightly compressed within the catheter and impose significant forces against the surrounding catheter bodies, often leading to excess friction between the graft and the catheter wall. These forces complicate the loading of the graft into the catheter, as well as the accurate release of grafts and stents in body lumens. Moreover, the catheters must maneuver the graft within the vascular system. Thus, the catheters are required to have flexible, elongate bodies which are particularly susceptible to the expanding graft, often resulting in invagination of the graft in the soft material of the catheter wall.

For these reasons, it would be desirable to provide improved apparatus and methods for endovascular placement of intraluminal protheses, including grafts, stents, and stent-grafts, for treating aneurysms and other conditions.

It would be particularly desirable to provide delivery catheters and methods for the placement of endoluminal tubular prostheses which would facilitate the controlled release of resilient tubular prostheses. It would be particularly desirable to provide delivery catheters and methods for the placement of endoluminal and other tubular prostheses which permit the repositioning and/or retrieval of partially released prostheses. It would be further desirable if such delivery catheters were able to contain the protheses firmly within the catheter until the final release of the prostheses into the blood vessel. It would also be particularly desirable to provide delivery catheters and methods which reduce the frictional forces created by the resilient expansion against the catheter during loading and release of the prostheses.

### 2. Description of the Background Art

Vascular grafts and devices for their endoluminal placement are described in U.S. Patent Nos. 5,282,824; 5,272,971; 5,242,399; 5,219,355; 5,211,658; 5,201,757; 5,192,297; 5,190,058; 5,158,548; 5,147,370; 5,104,399; 5,092,877; 5,078,726; 5,019,085; 4,990,151; 4,950,227; 4,913,141; 4,886,062; 4,820,298; 4,787,899; 4,617,932; 4,562,596; 4,577,631; and 4,140,126; and European Patent Publications 539,237; 533,511; 518,839; 518,704; 508 473; 505,686; 466 518; and 461 791. Catheters for placing vascular stents are described in U.S. Patent Nos. 5,192,297; 5,092,877; 5,089,005; 5,037,427; 4,969,890; and 4,886,062. Catheters carding a graft structure in a tube or capsule are described in U.S. Patent Nos. 5,275,622; 5,104,399; and 4,787,899; and EP466518.

### SUMMARY OF THE INVENTION

The present invention provides apparatus and methods for the endoluminal placement of intraluminal prostheses, including grafts, stents, and stent-grafts, for the treatment of disease conditions, particularly aneurysms. The intraluminal prostheses will typically comprise a resilient, radially compressible, tubular frame having a proximal end, a distal end, and an axial lumen therebetween. In the case of graft prostheses, a liner, typically a fabric, polymeric sheet, membrane, or the like, will line all or most of the luminal surface of the tubular frame, usually extending from a near-proximal location to a near-distal location. Suitable graft structures for placement using the catheters and methods of the present invention are described in copending application Serial No. 08/255,681, the full disclosure of which is incorporated herein by reference.

The intraluminal prostheses of the present invention are suitable for a wide variety of therapeutic uses, including stenting of the ureter, urethra, biliary tract, and the like. The present devices and methods will also be useful for the creation of temporary or long term lumens, such as the formation of fistulas. The present invention will find its greatest use, however, in the placement of endovascular prostheses into blood vessels for the treatment of abdominal and other aneurysms, vascular stenoses, and the like.

According to the present invention, a delivery catheter for positioning a radially compressible tubular prosthesis comprises an elongate flexible shaft having a proximal end and a distal end. A retaining structure is disposed near the distal end of the shaft and releasably holds the prosthesis to maintain the axial position of the prosthesis on the shaft. A sheath is slidably received over the shaft and radially compresses the prothesis while the prosthesis remains axially held by the retaining structure. Alternatively, the retaining structure can comprise a separate cover structure which maintains radial compression of the prostheses as the sheath is proximally retracted.

The prosthesis can be partially released from the catheter into a blood vessel or other body lumen by axially retracting the sheath to allow the prosthesis to expand and conform to the interior surface of the lumen being treated. The prosthesis, however, will remain attached to the catheter shaft by the retaining structure, and so long as the prosthesis remains attached, it can be recaptured simply by distally advancing the sheath back over the expanded portion of the prosthesis to radially compress it back on to the underlying shaft. In this way, the prosthesis can be recaptured and optionally repositioned and rereleased. Alternatively, the prosthesis can be withdrawn from the body lumen entirely.

In order to facilitate release and optional recapture of the prosthesis relative to the catheter, the sheath is preferably provided with a flared distal end. In a first particularly preferred design, a mechanism will be provided for reconfiguring the distal end of the sheath between a non-flared configuration (maintained during introduction of the catheter to a target location in the body lumen) and a flared configuration. For example, an inflatable bladder may be provided at the distal end, where inflation of the bladder causes the distal end of the sheath to flare radially outward. Alternatively, the distal end may include a resilient structure having a fixed, outwardly flared configuration. During introduction of the catheter to the body lumen, the resilient flared end may be radially confined, *e.g*., by a slidable containment sleeve disposed over the sheath or by an axially translatable cap structure which can capture the flared end of the sheath. As yet another alternative, the distal structure of the sheath may comprise heat memory alloy components which remained non-flared at low temperatures (to facilitate introduction) but which assume the desired flared structure when introduced to a body temperature environment.

As an alternative or in addition to the flared distal end of the prosthesis-containment sheath, the catheter may be provided with a tubular membrane which is attached at one end to the shaft at a location proximal of the retaining structure and at the other end to the inside of the sheath. The membrane is thus disposed to envelope the prosthesis once a prosthesis is received in its radially compressed configuration within the sheath. As the sheath is proximally retracted, a radially outward portion of the tubular member is drawn backwards, causing the tubular member to evert to release the expanding prosthesis while continuing to cover the portion of the prosthesis which is being released from the sheath. By providing a tubular membrane formed from a lubricous material, release and optional recapture of the radially compressible prosthesis can be greatly facilitated.

The retaining structure on the catheter shaft which anchors the prosthesis can take a variety of forms. Most simply, the retaining structure can comprise a plurality of locking stays which extend radially outward to penetrate the prosthesis and engage the interior wall of the sheath. Such locking stays are preferably circumferentially spaced-apart over the region of the catheter shaft which is axially aligned near the proximal end of the prosthesis when held on the shaft. In this way, the prosthesis will be held in place as the containment sheath is proximally translated in order to release the prosthesis. As a particular advantage, the prosthesis will also be held firmly in place if it is desired to distally advance the sheath in order to recapture the partially expanded prosthesis.

In an alternative configuration, the retaining structure comprises a pair of axially-spaced-apart locking stays and a pull wire which passes through each of the locking stays. By further passing the pull wire through the radially compressible prosthesis, the prosthesis may be held in place on the catheter shaft until the pull wire is removed. A wide variety of other specific mechanisms for retaining the prosthesis on the catheter shaft are also available.

The retaining structure may also comprise a cover which is detachably secured over the radially compressible prosthesis where the delivery catheter further includes a mechanism operable from the proximal end of the flexible shaft for detaching the cover to release the prothesis. Usually, the covered prosthesis will be further contained within the axially slidable sheath so that the sheath is first retracted to expose the cover, and the cover is then released from the radially expansible prosthesis within the blood vessel or other body lumen.

The detachable cover may comprise a variety of structures and/or mechanisms but will usually be a cylindrical structure encased around the prosthesis. Exemplary covers and detachment mechanisms include a cord which may be drawn proximally in order to split the cylinder along at least one axial or spiral line. Alternatively, the cover can comprise a plurality of resilient, radially flared axial elements which are held in place by an axially translatable end cap. The end cap may be selectively distally advanced in order to release the axial elements from around the prosthesis. As further a alternative, the cover may be axially weakened along a circumferential or helical line, where a proximal portion of the cover may be drawn in a proximal direction in order to separate the cover into two halves which release the prosthesis from therebetween.

In yet another aspect of the delivery catheter of the present invention, a journal sleeve may be slidably disposed over the prosthesis-containing sheath in order to permit external anchoring of the catheter while the sheath remains free to axially translate relative to the journal sleeve and shaft. Preferably, a lock will be provided to selectively attach the shaft to the journal sleeve while permitting the sheath to be axially translated. In this way, the position of the catheter shaft and radially compressed prosthesis (which is held thereon) may be fixed within the body lumen and then locked into place by locking the journal sleeve within an introducer sheath or other access device which is provided for percutaneous access. After the shaft and prothesis are locked in place, the sheath may be proximally retracted in order to release the prosthesis (allowing it to radially expand) without disturbing the preset position of the prosthesis within the lumen.

In yet another preferred aspect of the catheter introducing system of the present invention, a prosthesis cartridge for use with a delivery catheter comprises (1) a shaft extension having a proximal end, a distal end, and a coupling member at the proximal end, and (2) a sheath extension having a proximal end, a distal end, and a coupling member at the proximal end. A prosthesis is radially compressed over the shaft extension and within the sheath extension. The prosthesis cartridge can be connected to a delivery catheter including an elongate flexible shaft having a proximal end, a distal end, and a coupling member at the distal end for mating with the coupling member on the shaft extension. The delivery catheter will further include an elongate member slidably attached to the shaft and having a proximal end, a distal end, and a coupling member at the distal end for mating with the coupling member on the sheath extension. By providing prosthesis cartridges having a variety of useful lengths and/or diameters, the need for maintaining a large inventory of delivery catheters can be greatly reduced. Moreover, having an available inventory of precompressed prostheses, such as vascular grafts or stents, which are maintained sterilely greatly facilitates use of the delivery catheter system.

According to the method of the present invention, a shaft having a radially compressed prosthesis on its distal end is positioned at a target location within a body lumen, such as a blood vessel. A sheath which is disposed over the prosthesis to maintain its radial compression is then retracted in a proximal direction to permit radial expansion of the prosthesis while the prosthesis remains axially anchored on the shaft. Optionally, the sheath may be distally advanced to recompress and recapture the prosthesis at any time while the prosthesis remains anchored to the shaft. After the sheath has been substantially entirely retracted over the prosthesis, the prosthesis is released from the shaft to effect implantation. Preferably, both the shaft sheath are percutaneously introduced to the body lumen.

In a preferred aspect, the method of the present invention further comprises flaring a distal end of the sheath to facilitate retraction and optional advancement of the sheath over the prosthesis. The sheath may be flared by any of the mechanisms described above in connection with the apparatus of the present invention. Alternatively, the method can further comprise everting a tubular membrane which covers the prosthesis within the sheath as the sheath is proximally retracted. The membrane prevents direct contact between the sheath and the prosthesis and thus facilitates release and recapture.

Additionally, according to the present invention, a delivery catheter for positioning a radially compressible prosthesis comprises an elongate flexible shaft structure having a proximal end and a distal end. The shaft structure includes a prosthesis receptacle near the distal end. A tubular cover is slidably disposed about the shaft with at least one runner disposed within the distal end of the cover, wherein the runner is formed of a harder material than is the cover. The prosthesis can slide against the hard runner material within the cover in response to a distal force applied from the shaft. Advantageously, the hardness of the runner material avoids invagination of the compressed prosthesis frame in the cover while allowing use of a softer, more flexible cover material to facilitate intraluminal maneuvering of the catheter. Additionally, reduced friction between the prosthesis and cover also facilitates the precise positioning of the prosthesis by reducing the forces input at the proximal end and transmitted through the catheter body. The reduced friction of the present delivery catheter is also beneficial when it is necessary to recapture a partially deployed prothesis.

As used herein, a "prosthesis receptacle" is a structure or region along a shaft in or over which a radially compressible tubular prosthesis is carried during maneuvering of the shaft and prosthesis within a body lumen. The prosthesis receptacle may include a structure or portion at or near the distal end of the shaft which engages the prosthesis to effect its release--for example, a distal force imparting structure on the shaft that restrains proximal movement of the prosthesis as the cover slides proximally. Although the devices and methods of the present invention are illustrated with continuous shafts and covers for clarity, the principles of the present invention are fully compatible with an attachable prosthesis cartridge, as described herein. Similarly, catheter diameters may be reduced proximally of the prosthesis to facilitate intravascular maneuvering within the scope of the present invention

Preferably, the present catheter includes a plurality of axially disposed runners affixed together at their proximal ends, thereby reducing contact between the prosthesis and the soft inner surface of the cover. Optionally, the runners remain around the prothesis as the cover slides proximally during deployment. Alternatively, the cover and runners are withdrawn together as the prosthesis slides against the hard runners. Usually, the runners facilitate loading of the prosthesis by compressing the prosthesis as the runners and prosthesis slide together proximally into the cover. The runners are preferably formed of a high strength metal such as stainless steel, a stainless alloy, titanium, a titanium alloy, or a shape memory alloy such as Nitinol™, ideally being 304 or 316 stainless steel.

In a preferred embodiment, the present delivery catheter comprises an elongate flexible shaft and a tubular cover slidably disposed about the shaft. A plurality of elongate runners extend distally from the shaft, the runners having a hardness greater than the cover. The runners are radially constrained when the cover is distally extended over a prosthesis receptacle on the shaft, and are released by proximally retracting the cover. Advantageously, the runners of the present invention can be used to help compress the prosthesis and load it into the cover without damaging the prosthesis frame, either prior to insertion in the patient, or to facilitate recapture of a prosthesis which has been partially deployed within a body lumen.

Preferably, the shaft structure further comprises a core shaft with a guidewire lumen, the core shaft and cover providing an atraumatic distal tip to avoid injury during insertion. Optionally, the core shaft is attached to the shaft. Preferably, the core shaft can slide independently of the shaft, allowing retraction of a distal nosecone through the prosthesis prior to withdrawing the runners. This reduces the possibility of moving a partially deployed prosthesis by allowing manipulation of the nosecone within the prosthesis, rather than withdrawing both the nosecone and the surrounding runners simultaneously.

Optionally, the present catheter further includes a housing at the proximal end of the shaft with a mechanical advantage mechanism, preferably a linear screw, which further reduces the actuation forces and allows precise, gradual release of the prosthesis. A friction reducer tube may further be provided to facilitate withdrawing of the cover through an introducer sheath. The friction reducer tube is slidably disposed over the cover of the delivery catheter of the present invention, and includes a seal against the cover to provide hemostasis. The friction reducer tube is insertable within an introduction sheath, and allows movement of the delivery catheter with less friction than the introduction sheath, which must seal against a variety of invasive surgical devices. Optionally, a brace mechanically couples the proximal end of the shaft to the introduction sheath to prevent distal movement of the prosthesis or runners during deployment. Such a brace is particularly advantageous when a single surgeon is to manipulate the delivery catheter of the present invention.

The runners are again preferably formed of a high strength alloy. There are preferably between 1 and 20 runners, each runner being a strip which is longer than the prosthesis. The total width of all the runners is limited by the internal diameter of the cover, as the runners are usually affixed about the shaft side to side, and the runner/shaft assembly must slide within the cover. The runners will often be narrower than this, however, to allow the expanded prosthesis to anchor to the inner surface of the body lumen between the runners. The runners are each preferably in the range from .01 to .09 inch wide, and preferably between .001 and .02 inch thick.

In another aspect, the present invention provides an improved orientation indicating catheter for placement of an asymmetric prosthesis in a branching body lumen. The present orientation indicating catheter comprises an asymmetric marker on the shaft structure which indicates the rotational orientation of the prosthesis. Preferably, a branch axial marker on the shaft structure indicates the axial location of the prosthesis branch. These rotational and axial indicators prevent placement of the prosthesis branch below the branching body lumen, or crossing of the branches, either of which could reduce or even completely block flow through one branch of the body lumen system. Similarly, a position indicating catheter having a safety marker prevents placement of a secondary prosthesis too far within the branch of a branching prosthesis, reducing the danger of the tubular prosthesis folding over and blocking flow.

In yet another aspect, the present invention provides an expandable tip catheter for placement of a radially compressible prosthesis having a large diameter portion and a small diameter end, the catheter comprising an elongate shaft structure having a prosthesis receptacle near a distal end, and a cover slidably disposed about the shaft structure. The cover comprises a body portion and a resilient structure extending from the distal end of the body portion. The catheter restrains the prosthesis with the small diameter end of the prosthesis in the distal resilient structure of the cover, allowing the distal end of the catheter to have a smaller outer diameter than the body. Advantageously, the distal end may be advanced into a smaller body lumen branch, and the expandable structure can expand over a large diameter end of the prosthesis as the cover is retracted into a larger body lumen. The resilient structure preferably comprises a braided mesh tubing with an elastomeric material disposed over the mesh tubing. Alternatively, the resilient structure comprises a sheath having one or more axial slits, or a rolled or folded pliable material.

According to the method of the present invention, a resilient, radially compressible prosthesis is loaded into a tubular cover by compressing the prosthesis between a plurality of elongate runners and sliding the runners into the cover. The cover may then be positioned within a body lumen so the prosthesis is at a target location, and deployed by withdrawing the cover relative to the prosthesis. Optionally, the runners exit the cover distally with the prosthesis, and are retracted after the cover. Alternatively, the runners and cover are retracted together.

### BRIEF DESCRIPTION OF THE DRAWINGS

Fig. 1 is a side view of a vascular graft which is exemplary of the type of radially compressible tubular prosthesis which may be placed using the delivery catheter of the present invention.
Fig. 2 is a perspective view of a first embodiment of a delivery catheter of the present invention.
Figs. 3-5 illustrate the use of the delivery catheter of Fig. 2 in placement of a radially compressible tubular prosthesis in a body lumen.
Fig. 6 illustrates placement of a journal sleeve at the proximal end of the delivery catheter of Fig. 2.
Figs. 7 and 8 illustrate a second embodiment of the delivery catheter of the present invention, wherein a prosthesis-containing sheath has a flared distal end which is open and closed by a sliding sleeve.
Figs. 9 and 10 illustrate a third embodiment of the delivery catheter of the present invention wherein the prosthesis-containing sheath has a distal end which may be flared by inflating a bladder surrounding its distal end.
Fig. 11 illustrates a delivery catheter sheath having a flared end including heat memory alloy components.
Fig. 12 illustrates a delivery catheter sheath having a flared distal end which is contained within an axially translatable cap.
Fig. 13-15 illustrate a fourth embodiment of the delivery catheter of the present invention having an eversible membrane for containing a radially compressible prosthesis.
Fig. 16 illustrates a delivery catheter wherein a prosthesis is anchored to the catheter shaft by a pull wire.
Fig. 17 and 18 illustrate a prosthesis cartridge constructed in accordance with the principles of the present invention.
Figs. 19A-19D illustrate a delivery catheter constructed in accordance with the principles of the present invention which includes a retaining structure comprising a pair of axially spaced-apart anchor members to hold the prosthesis in place as the sheath is drawn proximally from over the prosthesis.
Figs. 20A and 20B illustrate an alternative embodiment of a prosthesis cover structure according to the present invention, where the cover is a cylinder having a weakened line disposed helically over its surface. The cover may be opened by pulling proximally on one end of the cylinder.
Figs. 21A and 21B illustrate another alternative embodiment of the prosthesis cover structure of the present invention, where a tear wire is attached to a pull cord for opening the cover along a helical line.
Figs. 22A and 22B illustrate yet another embodiment of the prosthesis cover structure of the present invention, where a single axial line or perforation may be opened using a zipper structure.
Figs. 23A and 23B illustrate still another embodiment of the prosthesis cover structure of the present invention, where a plurality of radially flared resilient elements are held by an end cap and may be released by distally advancing the end cap.
Figs. 24A-24C illustrate yet another alternative embodiment of the prosthesis cover structure of the present invention, where the cover is weakened along a circumferential line, permitting the cover to be drawn axially apart to release the prosthesis.
Fig. 25 is a perspective view of another embodiment of a delivery catheter of the present invention, with a portion of the distal end broken away to disclose a prosthesis therein.
Figs. 26A and 26B illustrate the loading of a graft into the delivery catheter of Fig. 25.
Figs. 27A-27C illustrate the use of the delivery catheter of Fig. 25 in placement of a radially compressible tubular prosthesis in a body lumen.
Fig. 28 illustrates a preferred method of use of the delivery catheter of Fig. 25, in which tapered nosecone is withdrawn independently of the runners.
Fig. 29A is an exploded cross-sectional view of the delivery catheter of Fig. 25.
Fig. 29B is a cross-section of an alternative shaft structure and cover having increased flexibility.
Fig. 30 illustrates a housing at the proximal end of the delivery catheter of Fig. 25 which provides a mechanical advantage for withdrawing the cover.
Fig. 31 illustrates a delivery catheter cover having a rounded, atraumatic distal end with a split tip.
Fig. 32 illustrates a delivery catheter cover having runners imbedded within the distal end.
Figs. 33A and 33B are alternative cross-sectional views of a delivery catheter cover.
Fig. 34 is a side view of a vascular graft which is exemplary of the branching, resilient, radially compressible prostheses which may be placed in branching body lumens using the delivery catheters of the present invention.
Figs. 35A and 35B illustrate a preferred embodiment of an alternate embodiment of the present delivery catheter having a cover with an expandable distal structure for use with the branched stent of Fig. 34.
Figs. 36A-36D are alternative cross-sectional views of the expandable structure of the delivery catheter of Figs. 35A and 35B.
Figs. 37A-37C illustrate the use of the alternative delivery catheter of Figs. 35A and 35B.
Fig. 38 illustrates a core shaft having a preferred nosecone for use with the delivery catheters of the present invention to rotationally and axially position branching prostheses within branching body lumens under imaging.
Figs. 39A-39E illustrate the use of a delivery catheter having the preferred shaft of Fig. 38 in placement of a branched prosthesis within a branching body lumen.
Fig. 40 is a cross-sectional view of a friction reducer tube disposed over the cover of the present delivery catheter.
Fig. 41 illustrates the use of the friction reducer tube of Fig. 40 to reduce the friction of an introduction sheath valve during placement prostheses with the present delivery catheters.
Fig. 42 illustrates a brace which restrains the prosthesis at a target location during deployment.

### DESCRIPTION OF THE PREFERRED EMBODIMENT

The present invention provides apparatus and methods for the endoluminal placement of intraluminal tubular prostheses, particularly grafts, stents, and stent-grafts. The tubular prostheses will be radially compressible, and the apparatus of the present invention will maintain the prostheses under compression in a narrow-diameter configuration while they are being introduced to the body lumen, typically during surgical cutdown or percutaneous introduction procedures. Placement of the tubular prosthesis is effected by releasing the prosthesis at a target location in the lumen. Thus, it is necessary that the prosthesis be sufficiently resilient and conformable to expand against the interior wall of the body lumen. It will be appreciated, however, that the prosthesis may be formed at least partly from malleable components which permit it to be subsequently further expanded, typically by inflation of a balloon within the lumen of the prosthesis.

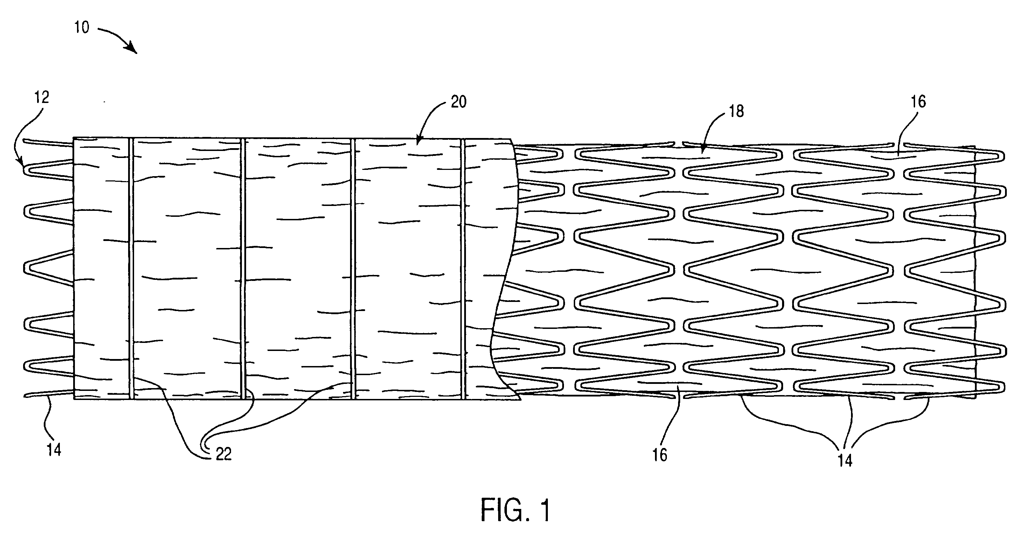

The present invention will find greatest use in the percutaneous placement of endovascular prostheses for the treatment of diseases of the vasculature, particularly aneurysms, stenoses, and the like. Suitable prosthesis structures which may be deployed by the delivery catheter of the present invention are described in copending application Serial No. 08/255,681, the full disclosure of which is incorporated herein by reference. One exemplary graft structure 10 is illustrated in Fig. 1. Prosthesis 10 comprises a perforate tubular frame 12 which includes a plurality of independent (non-connected) band members 14 separated from each other by small gaps 16. The tubular frame 12 is covered by an inner liner 18 and an outer liner 20, where the inner and outer liners together encase or sandwich the otherwise free-floating band members 14 therebetween. In order to secure the band members 14 in place, and secure the liners to the perforate tubular frame 12, the inner and outer liners are joined together along circumferential lines 22, preferably aligned with the gaps 16 between adjacent band members 14. The liners may be joined together by stitching, heat welding, ultrasonic welding, or the like. In the exemplary embodiment, the liners 18 and 20 are formed from polymeric sheet material and are joined together by ultrasonic welding. The band members 14 at each end of the graft 10 will have to be further secured to the liners 18 and 20. For example, they could be stitched, welded, or otherwise joined to the liners to hold them in place. The graft 10 will typically have a length in the range from about 50 mm to 500 mm, preferably from 80 mm to 200 mm, with a relaxed diameter in the range from about 4 mm to 45 mm, preferably being in the range from 5 mm to 25 mm. Such graft structures will be particularly suitable for treating vascular aneurysms.

In connection with the present invention, it has been discovered that the placement of resilient tubular prostheses imposes serious demands on delivery and imaging systems, as well as on the attending medical personnel. Prostheses are highly compressed within delivery catheters to allow maneuvering within the vascular system. The compressive forces have been found to lead to excessive friction during deployment from the delivery catheters of the prior art. Additionally, visualization of compressed prostheses within the catheter is problematic, particularly when a branched prosthesis must be placed in a branching body lumen in a specific orientation.

The delivery catheters of the present invention facilitate deployment of resilient prostheses by reducing friction at the prosthesis/catheter interface, avoiding any increase in the stiffness of the delivery system where it is not needed. In connection with the present invention, it has been discovered that compressed prostheses are largely rigid, which reduces any penalty in flexibility imposed by including hard, friction-reducing runners around the prosthesis.

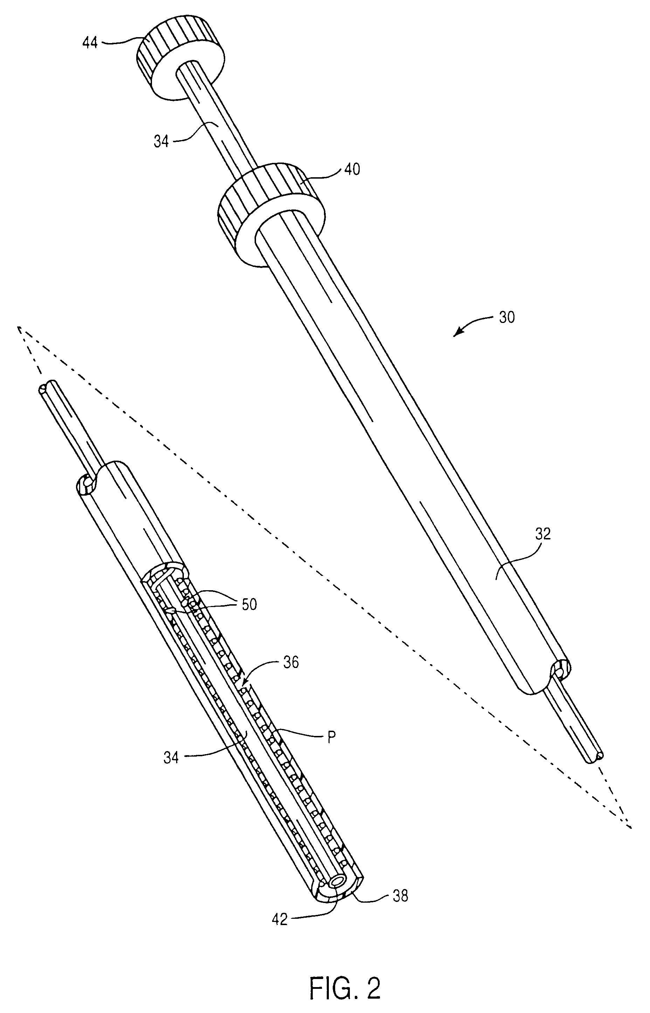

Referring now to Fig. 2, a delivery catheter 30 constructed in accordance with the principles of the present invention comprises a sheath 32 and a shaft or inner catheter body 34. The sheath 32 has a central lumen 36 extending from a distal end 38 to a proximal handle 40. The shaft 34 is slidably received within the central lumen 36 and has a distal end 42 and a proximal handle 44. The delivery catheter 30 receives a radially compressible tubular prosthesis P within the annular space between the outer surface of the shaft 34 and the inner surface of the lumen through sheath 32. For convenience, the prosthesis is illustrated as a radially compressed helical coil which expands by unwinding and axial shortening. The delivery catheters of the present invention, however, can be used with virtually any radially compressible prosthesis, as described above.

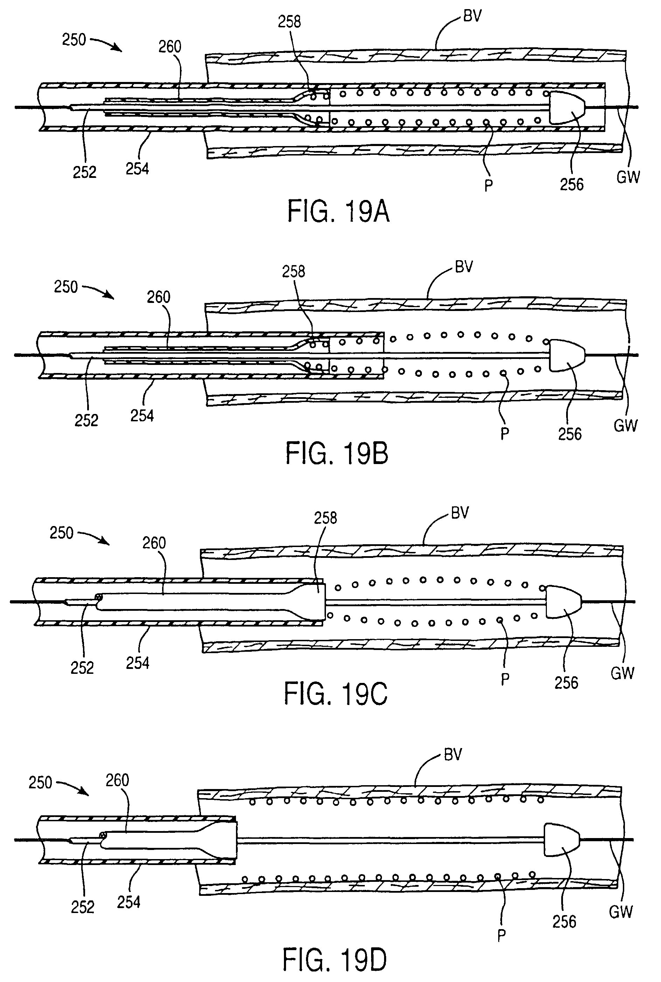

The delivery catheter of Fig. 2 relies on maintaining the radial compression of prosthesis P by direct pressure from the sheath 32. As will be discussed in detail below in connection with Figs. 19-24, prosthesis compression may also be provided by a retaining structure which comprises a cover, spaced-apart anchors, or other equivalent structure which maintains the radial compression regardless of the position of the sheath. Using such embodiments, the prosthesis may be uncovered and located prior to release and radial expansion.

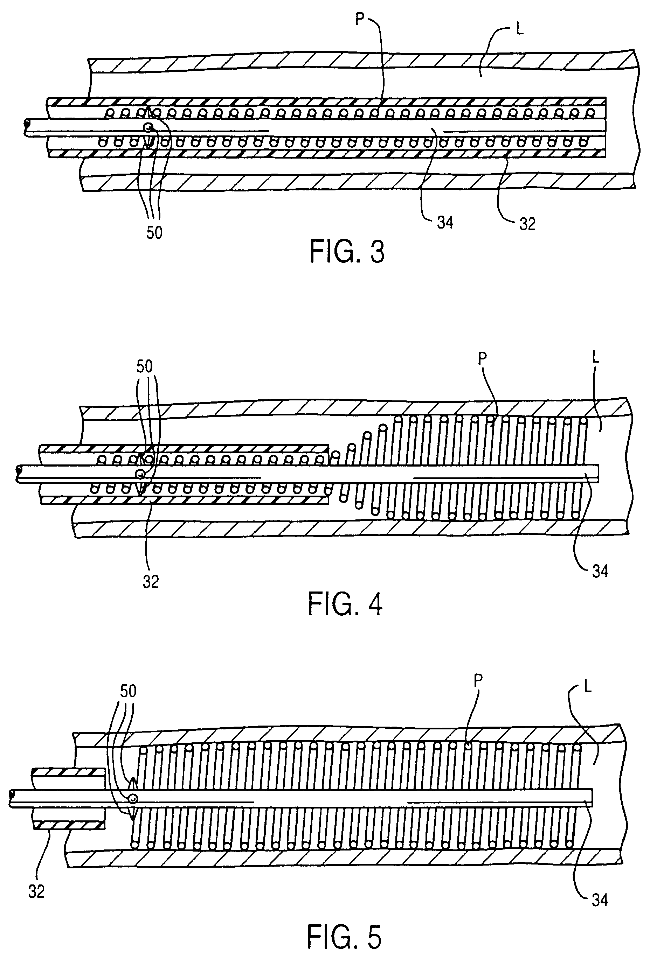

In the embodiment of Fig. 2, the prosthesis P is anchored by a plurality of penetrating stay members 50 which are circumferentially spaced-apart over the exterior of the shaft 34. The stays 50 will be spaced proximally from the distal end 42 of the shaft 34 by a distance which corresponds generally to that of the tubular prosthesis P which is to be maintained on the delivery catheter 30. The penetrating stays 50 will extend radially outward by a distance sufficient to engage the interior surface of the lumen 36 of the sheath 32. In that way, the penetrating stays 50 will be able to anchor the proximal end of the tubular prothesis P when it is held within the catheter. In particular, the prosthesis P will remain anchored as the sheath 32 is drawn proximally over the shaft 34, as illustrated in Figs. 3-5.

When initially placed in a body lumen L, the sheath 32 covers substantially the entire length of the prosthesis P with the penetrating stays 50 engaging the proximal portion of the prosthesis P, as illustrated in Fig. 3. The sheath 32 may then be retracted proximally, partially releasing the prosthesis P, as illustrated in Fig. 4. The proximal portion of the prosthesis P, however, remains anchored by the penetrating stays 50 so long as the sheath 32 remains positioned over the stays. Once the sheath 32 is withdrawn to the proximal side of the stays 50, as illustrated in Fig. 5, the prosthesis P will be fully released. Prior to such full release, however, the prosthesis P may be recaptured by advancing the sheath 32 in the distal direction relative to the shaft 32.

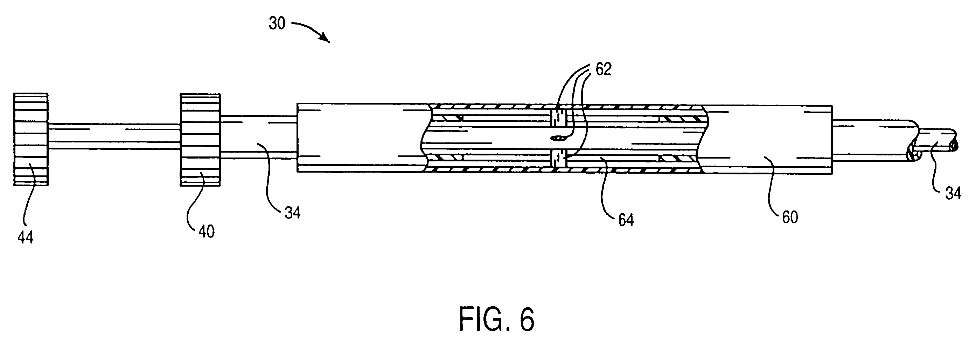

Referring now to Fig. 6, the catheter 30 may optionally be provided with a journal sleeve 60 near its proximal end. The journal sleeve 60 is preferably mechanically coupled to the shaft 34 by pins 62 which extend through slots 64 in the sheath 32. The journal sleeve 60 can be anchored within an introducer sleeve or other access device (not illustrated) which is used to provide percutaneous access to the body lumen being treated. After initial positioning of the catheter 30 so that the prosthesis P is located at the target location within the lumen, it is desirable to firmly anchor the catheter 30 within the introducer sheath. Journal sleeve 60 permits anchoring of the shaft 34 (which carries the prosthesis P) while allowing the sheath 34 to remain freely translatable relative to both the journal sleeve 60 and the catheter shaft 34.

The dimensions and materials of construction of the catheter 30 may vary widely, depending on the intended usage. For vascular applications, the catheter 30 will typically have a length in the range from about 50 cm to 250 cm, preferably from 100 cm to 200 cm, and a diameter in the length from about 3 mm to 8 mm, preferably from 4 mm to 6 mm. These dimensions generally refer to the exterior dimensions of the sheath 32. It will be appreciated that the catheter shaft 34 will have a smaller diameter, typically in the range from 1 mm to 5 mm, preferably from about 1.5 mm to 3 mm, allowing a sufficient annular space therebetween to receive the prosthesis P. The catheter shaft will also have a length which is greater than that of the sheath, usually by a distance sufficient to accommodate the length of the prosthesis which is being delivered, typically from 5 cm to 25 cm, preferably from 7.5 cm to 15 cm. The catheters will generally be constructed of natural or synthetic polymers, such as silicone rubber, natural rubber, polyvinylchloride, polyurethanes, polyesters, polyethylenes, polytetrafluoro-ethylenes (PTFE), and the like. Optionally, the catheter sheath and shaft may be formed as composites having a reinforcement layer incorporated within a polymeric body in order to enhance strength, flexibility, and toughness. Suitable reinforcement layers include wire mesh layers, braided layers, and the like. The tubular members of the present invention may be formed by extrusion, with the tubular diameter modified by heat expansion and/or shrinkage using conventional techniques. Particular techniques for forming vascular and other catheters suitable for use in the present invention are well described in the patent and medical literature.

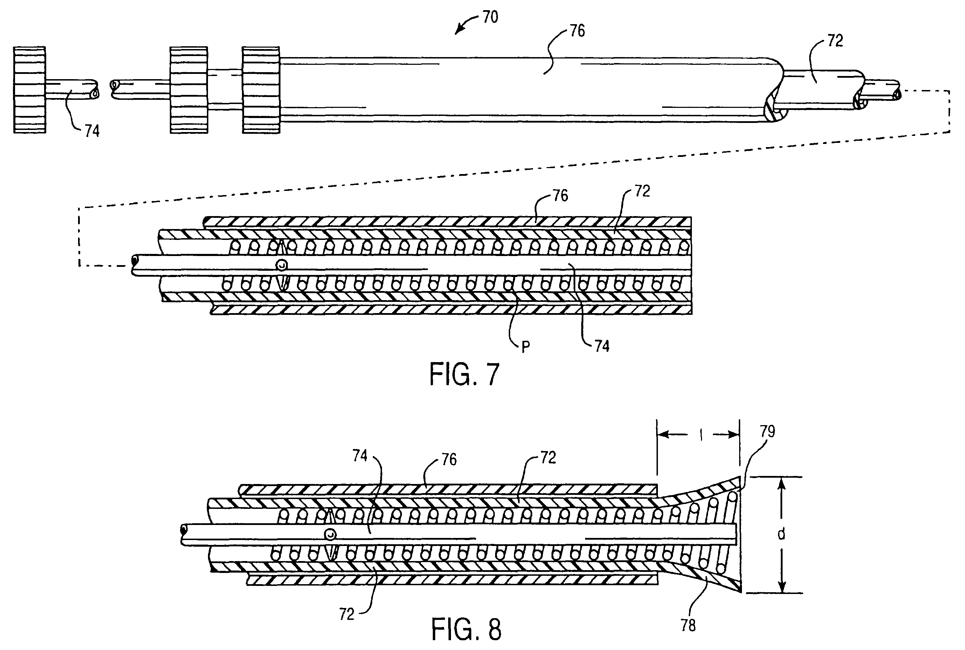

Referring now to Figs. 7 and 8, a catheter 70 having a sheath 72 with a deployable flared end will be described. Catheter 70 comprises the sheath 72, a shaft 74, and a prosthesis-containment sheath 76. A prosthesis P is contained between the sheath 72 and the shaft 74, generally as described above in connection with delivery catheter 30. The sheath 72, however, differs from that of sheath 32 in that sheath 72 has an outwardly flared distal end 78, as best seen in Fig. 8. The distal end 78 is a resilient structure, typically formed from the material of the sheath 72 itself and optionally having a plurality of elastic reinforcement elements imbedded therein to maintain the desired flared configuration, and may be radially collapsed by the containment sleeve 76, as illustrated in Fig. 7. The flared distal end of the sheath 72 is advantageous since it facilitates the release and recapture of the prosthesis P.

The flared distal end 78 of catheter 70 will usually have a fully expanded diameter **d** at the distal tip 79 in the range from 10 mm to 30 mm, preferably from 15 mm to 25 mm. The distal tip of diameter **d** will usually be greater than the diameter of the proximal portions of the sheath 72 by a factor from 2 to 8, preferably being from 2.5 to 5. The flare will extend over an axial length ℓ in the range from 3 mm to 30 mm, preferably from 5 mm to 20 mm. These flare dimensions will generally be applicable to all embodiments of the present invention where the prosthesis-containment sheath has a flared distal end.

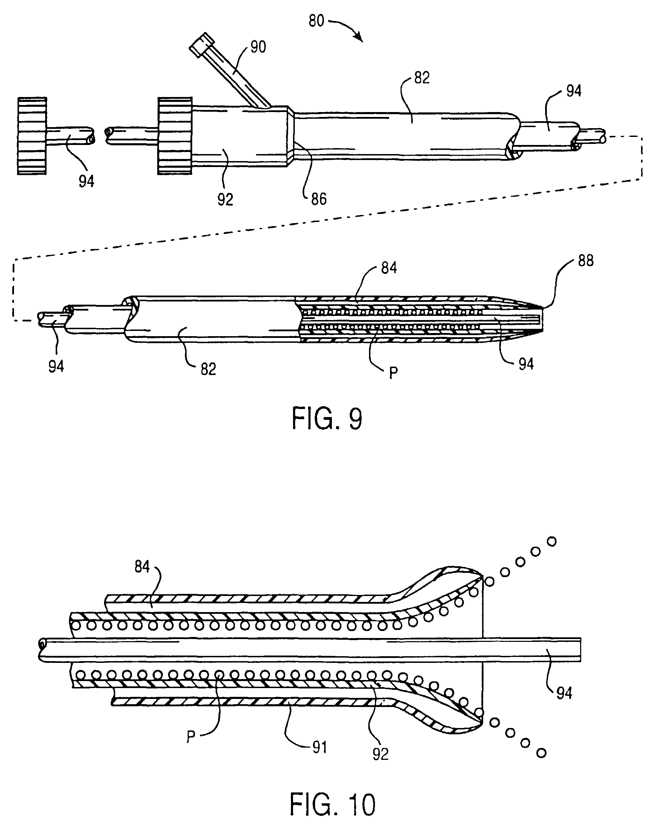

Referring now to Figs. 9 and 10, a catheter 80 having an alternate mechanism for deploying a flared distal tip on a prosthesis-containing sheath structure 82 will be described. Catheter 80 comprises the sheath 82 having an annular lumen 84 extending from its proximal end 86 to its distal end 88. The annular lumen 84 is connected to an inflation port 90 on a proximal housing 92. A shaft 94 extends through the central lumen of the sheath 82 and carries a prosthesis P near it distal end.

The distal end of the sheath 82 is formed so that, upon inflation with a non-compressible fluid medium, typically saline or other biocompatible liquid, it assumes the outwardly flared configuration shown in Fig. 10. The structure is sufficiently elastic, however, so that removal of the inflation medium will permit the sheath 82 to resume its non-flared configuration, as illustrated in Fig. 9. Flaring of the distal end of sheath 82 facilitates both release and recapture of the prosthesis P, as with the embodiment of Figs. 7 and 8.

Conveniently, distal end 88 of sheath structure 82 comprises an outer layer 91 secured to an inner layer 92 at their respective distal ends. Both layers 91 and 92 will be composed of a flexible, non-distendable material, such as polyethylene terephthalate (PET), or other reinforced material, such as an elastomeric or non-elastomeric material reinforced with a non-distendable mesh. The outer layer will be shorter than the inner layer so that when the annular lumen 84 is inflated, the distal end will flare as shown in Fig. 10.

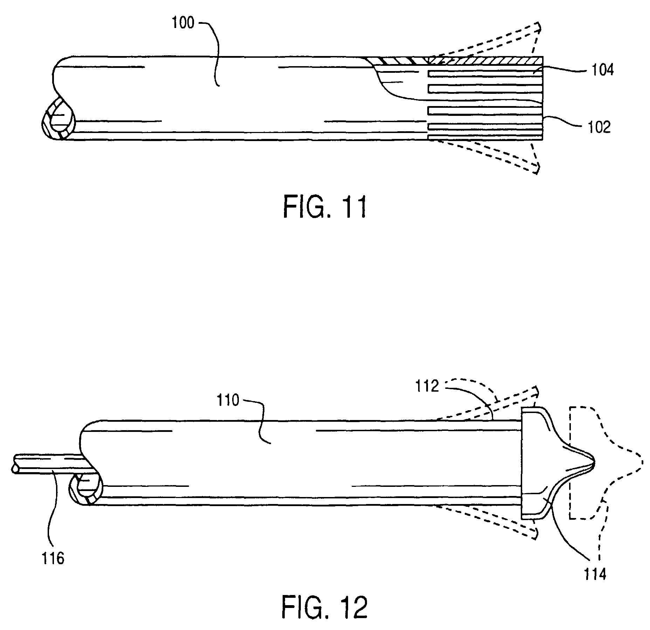

Alternative mechanisms for providing a deployable flare at the distal end of a prosthesis-containment sheath are illustrated in Figs. 11 and 12. The sheath 100 in Fig. 11 has a distal end 102 including a plurality of axially aligned, circumferentially spaced-apart heat memory alloy members 104. The heat memory alloys are selected to have a temperature transition where they assume a straight, non-flared configuration at low temperatures, as illustrated in full line in Fig. 11. At body temperature, however, the members 104 assume an outwardly flared configuration, as illustrated in broken line. Suitable alloy materials include nickel-titanium alloys which may be heat treated to provide the proper shapes and transition temperature.

Sheath 110 illustrated in Fig. 12 has a resilient, flared structure formed at its distal end 112. The flared distal end 112 is contained in an end cap 114 which may be distally advanced (as illustrated in broken line) by shaft 116 to release the flared end structure, as shown in broken line.

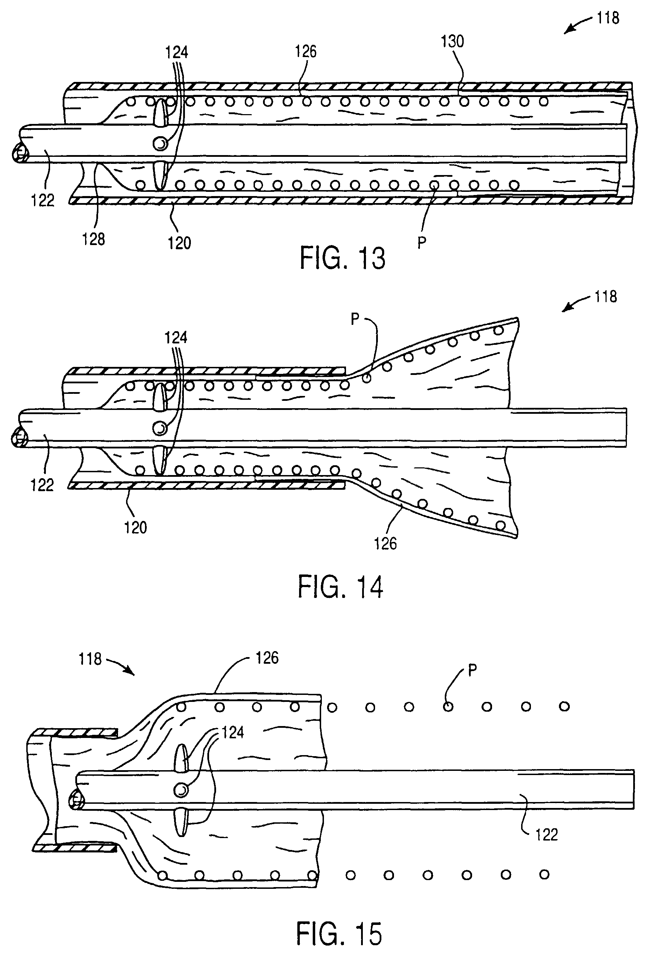

An alternative structure for facilitating the release and recapture of a prosthesis from a delivery catheter according to the present invention is illustrated in Figs. 13-15. A catheter 118 is provided with a sheath 120, shaft 122, and penetrating stays 124, generally as described above in connection with Figs. 2-5.

The catheter 118 further includes an eversible membrane 126 which is attached at a first end 128 to the shaft 122, and at a second end 130 to the inner surface of the lumen of sheath 120. The membrane 126 will be formed from a flexible, preferably lubricous and non-compliant material, such as PET, nylon, polytetrafluoroethylene (PTFE), any of which may be wire- or braid-reinforced, or the like. The prosthesis P will remain anchored on the shaft 122 by penetrating stays 124 as the sheath 120 is partially withdrawn (Fig. 14). The membrane 126 folds back over itself (everts) as the sheath 120 is retracted so that there are always two layers of the membrane between the distal end of the sheath and the prosthesis P. The double-layer structure of the membrane provides a high degree of lubricity during the release and optional recapture of the prosthesis P. Complete release of the prosthesis P is illustrated in Fig. 15.

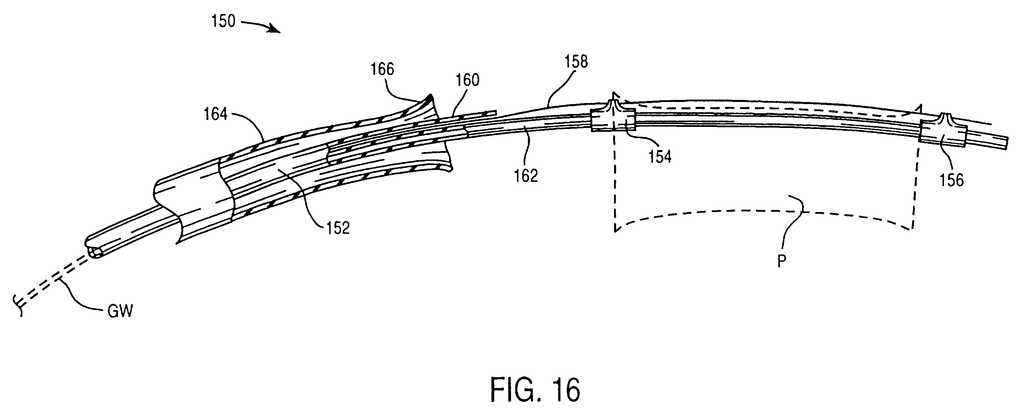

Referring now to Fig. 16, an alternative prosthesis anchoring mechanism for a delivery catheter 150 is illustrated. The delivery catheter 150 includes a shaft 152 having a pair of axially spaced-apart stays 154 and 156. A pull wire 158 extends through a lumen 160 of shaft 152 and through protrusions on each of the stays 154 and 156. A guide wire GW is received through the shaft 152 in order to permit vascular introduction by conventional techniques. The radially compressible prosthesis P (such as graft 10) is placed over the distal end of the shaft extension 162, generally being aligned between the stays 154 and 156. The pull wire 158 is then advanced through the stays 154 and 156 so that it passes through each end of the prosthesis P to maintain the prosthesis P in place until the pull wire is withdrawn. While the pull wire 158 remains in place, a prosthesis-containment sheath 164 may be axially advanced over the graft to radially compress the graft into its desired low profile diameter. The sheath 164 includes a flared (i.e., outwardly tapered) distal end 166 to facilitate advancing the sheath over the prosthesis P, in particular so that the prosthesis P may be recaptured when it is partially deployed. The outward flare may be permanently fixed in the body of the sheath, but will preferably be selectively deployable between the flared and non-flared configuration, using any of the mechanisms described above.

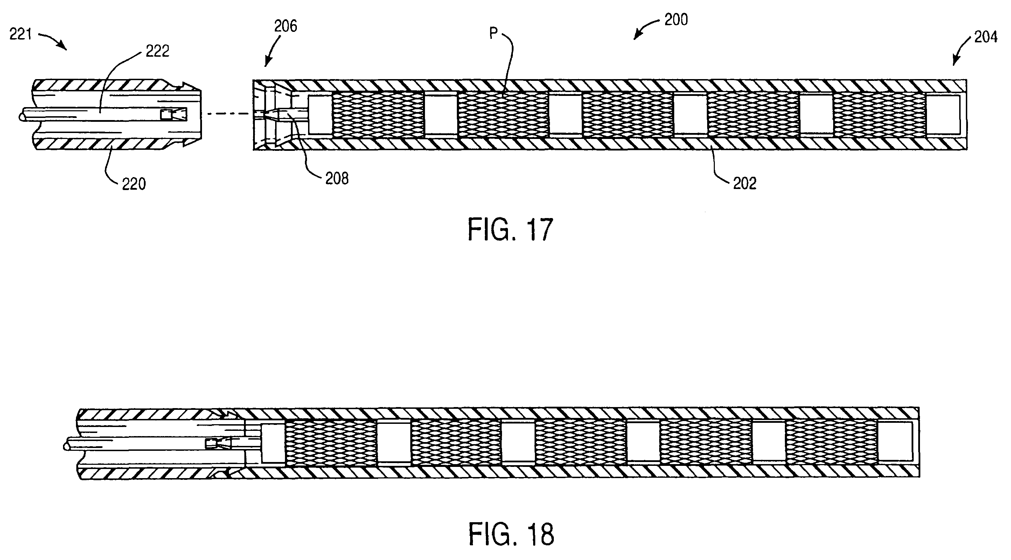

Referring now to Fig. 17 and 18, a prosthesis cartridge 200 comprises a sheath extension 202 having a distal end 204 and a proximal end 206. A prosthesis P is contained within the sheath extension 202 and is mounted over a shaft extension 208. Typically, the prosthesis P will be anchored on the shaft extension 208 using penetrating stays (not shown) as described in connection with previous embodiments. The prosthesis cartridge 200 is releasably connectable to a delivery catheter 221 including a sheath 220 (or other elongate member) and shaft 222. The proximal end of the cartridge sheath 202 is configured to couple to the distal end of the catheter sheath 220. Similarly, the proximal end of the shaft extension 208 is configured to selectively couple to the distal end of the shaft 222. In this way, a user can select the diameter, length, and other characteristics of the prosthesis P which are desired to be employed in a procedure. The prosthesis, which is part of cartridge 200 (and preferably packaged in a separate, sterile pouch or other container) may then be attached to the distal end of the delivery catheter (which is separately packaged in a sterile pouch or other container) having the necessary sheath and shaft connections. The catheter sheath 220 could alternatively comprise other, non-tubular structures (elongate members). It is necessary only that the elongate member be able to connect to the sheath extension 202 to be proximally retracted over the prothesis P (and optionally distally advanced) to effect release and recapture of the prosthesis as described above.

Referring now to Figs. 19A-19D, yet another embodiment of a delivery catheter 250 constructed in accordance with the principles of the present invention will be described. Delivery catheter 250 includes flexible shaft 252 having a central lumen for receiving a guide wire GW. A sheath 254 is slidably mounted over the shaft 252, generally as described for previous embodiments. The catheter 250 differs from previous embodiments, however, in the nature of the retaining structure which is used for holding prosthesis P in place on the flexible shaft 252. The retaining structure comprises a distal anchor 256, which is conveniently in the form of a cap or other receptacle which can receive a distal end of the prosthesis therein. A proximal anchor 258 is mounted at the distal end of a sliding tube 260. As shown in Fig. 19A, when the catheter 250 is introduced to blood vessel BV the prosthesis P will be maintained in its collapsed configuration by the anchors 256 and 258, and sheath 254 will cover the prothesis and anchor structures.

After introduction, as illustrated in Fig. 19B, the sheath 254 may be withdrawn proximally to expose the prosthesis P. The prosthesis P, however, remains radially compressed by the anchors 256 and 258, even after the sheath 254 has been fully withdrawn, as illustrated in Fig. 19C. The prosthesis P may be fully released by moving the anchors 256 and 218 axially apart in order to free the compressed ends of the prosthesis, as illustrated in Fig. 19D. Prior to release, however, the exposed prostheses can be carefully positioned without interference from the sheath 254. It is a particular advantage that such partial release is achieved while still being able to readily recapture the prosthesis by readvancing the sheath 254.

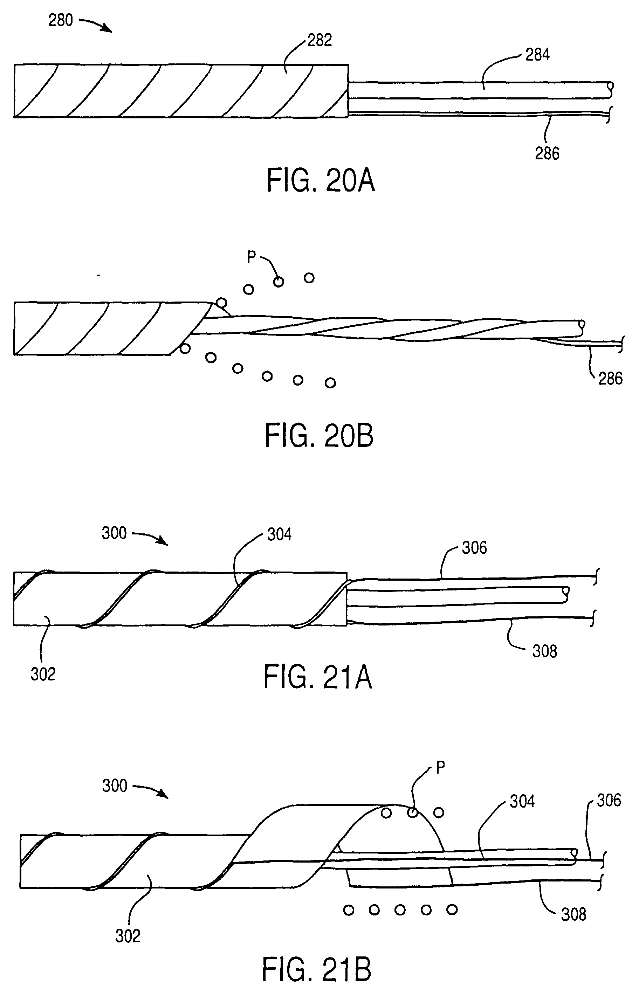

Referring now to Figs. 20A and 20B, an alternative embodiment of a prostheses retaining structure is illustrated. The retaining structure 280 will fully cover and compress the prostheses P, and will usually be maintained within an outer sheath (not shown) equivalent to the delivery catheter sheaths illustrated previously. The retaining structure 280 will maintain radial compression of the prosthesis P within the sheath, regardless of whether the sheath covers the prosthesis. Thus, the sheath of the associated delivery catheter may be proximally retracted prior to release of the prostheses P.

The retaining structure 280 comprises a helically wound ribbon, which may optionally be formed as a helically scored or perforated cylinder. The retaining structure 280 is mounted on flexible shaft 284, typically with a distal portion of the helical ribbon attached directly or indirectly to the shaft. A pull cord 286 is attached to a proximal end of the helical ribbon, and the ribbon may be withdrawn from over the prostheses P by pulling proximally on the pull cord, as illustrated in Fig. 20b.

Yet another embodiment of the retaining structure of the present invention is illustrated in Figs. 21A and 21B. Retaining structure 300 comprises a cylinder 302 having a helical wire 304 disposed over its surface. The wire 304, when pulled from the cylinder 302, separates adjacent sections of the cylinder so that they break apart, as illustrated in Fig. 21B. Thus, by attaching a first pull cord 306 to a proximal end of the wire 304, the wire can be withdrawn by pulling proximally. The resulting ribbon-like section of the cylinder may then be withdrawn by pulling on a second pull cord 308, also as shown in Fig. 21B. The prostheses P is thus released from the catheter.

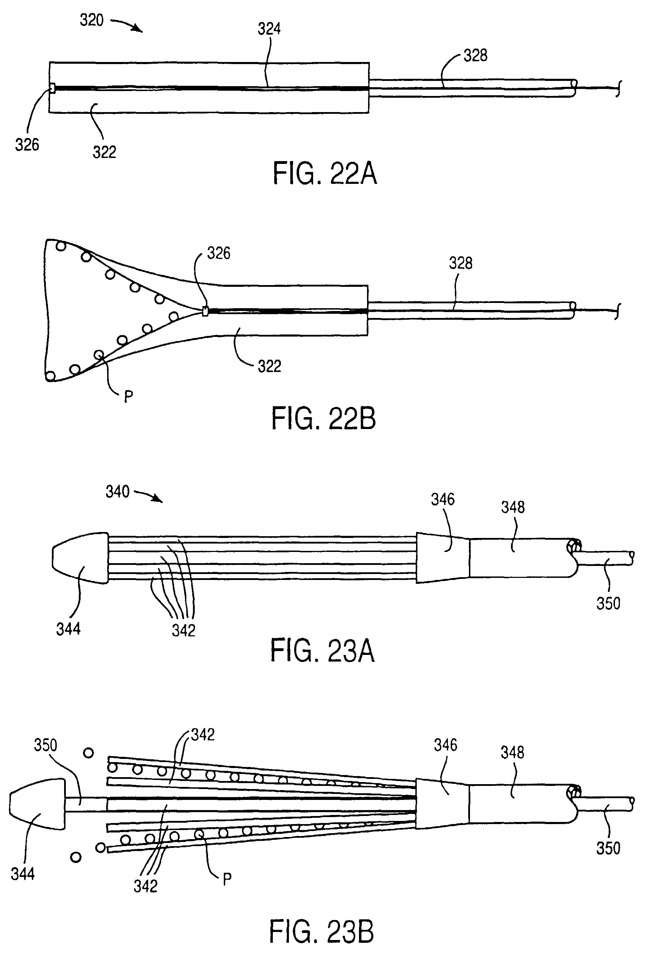

Yet another embodiment of a retaining structure of the present invention is illustrated in Figs. 22A and 22B structure 320 is a cylinder 322 having a single axial break line 324 formed along one side thereof. It will be appreciated that more than one axial break line may be provided, Only one is illustrated, however, for convenience. A slide structure 326 secured to the cylinder 322 at a distal end of the break line 324. A pull cord 328 is attached to the slide structure 326. Optionally, multiple pull cords could be used. The slide structure 326 may be drawn proximally in order to open the breakline 324 in the manner of a zipper, as illustrated in Fig. 22B. In this way, the prostheses P can be released.

Yet another embodiment of the retaining structure 340 of the present invention is illustrated in Figs. 23A and 23B. The retaining structure 340 comprises a plurality of individual resilient axial members 342 which are captured at their distal ends and an anchor 344. The axial elements 342 are permanently mounted in a ring structure 346 at the distal end of catheter body 348. The anchor 344 is secured at the distal end of a flexible shaft 350. The axial elements 342 are spring-loaded so that when the anchor 344 is moved distally by advancing the shaft 350, as illustrated in Fig. 23b, the individual elements will spring radially apart at the distal end. In this way, prosthesis P can be released from the retaining structure 340.

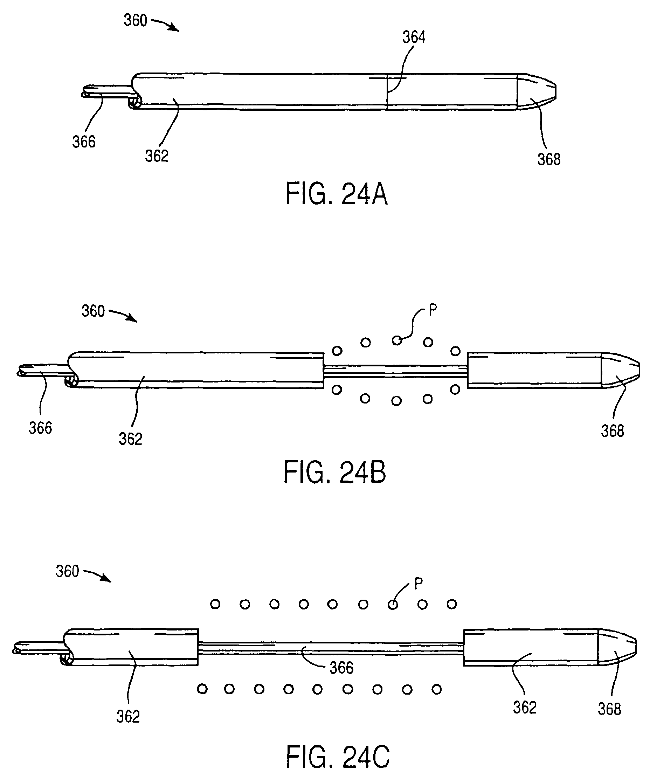

Referring now to Figs. 24A-24C, still another embodiment of retaining structure constructed in accordance with the principles of the present invention will be described. The retaining structure 360 is a thin-walled tube 362 which is weakened along a circumferential (or helical) line 364, typically in the form of a score, perforation, or the like. Flexible shaft 366 secured to a distal end cap 368. By axially advancing the shaft 366, the end cap 368 and the attached portion of cylinder 362 between the score line 364 and the end cap will be pulled away from the remainder of the cylinder 362. In this way, the prostheses P can be released. The prostheses is first partially released, as shown in Fig. 24B. After the cylinder segments are fully spaced-apart, the prostheses is fully released, as shown in Fig. 24C.

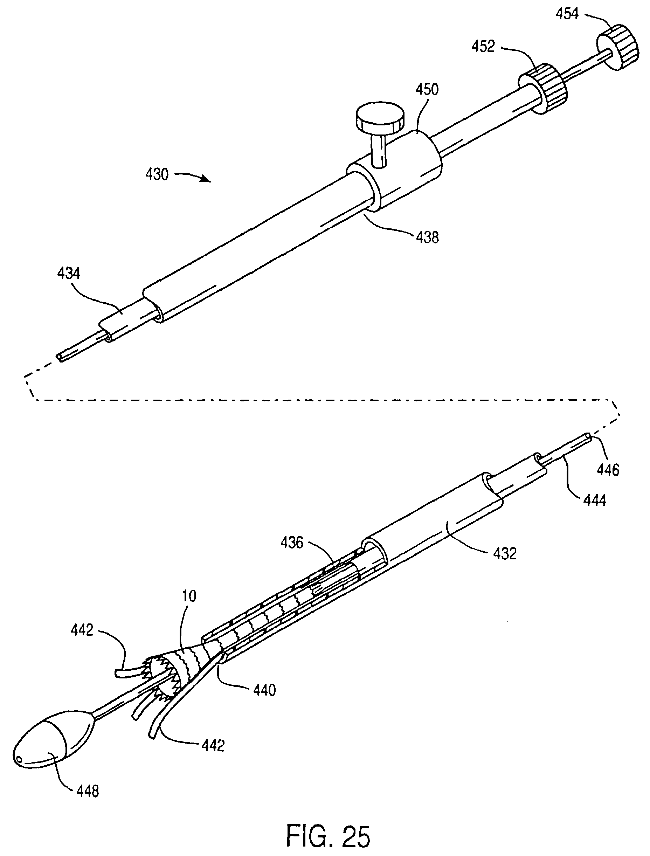

Referring now to Fig. 25, a delivery catheter 430 constructed in accordance with the principles of the present invention comprises a tubular cover 432 and a shaft or inner catheter body 434. Cover 432 has a central lumen 36 extending from a proximal end 438 to a distal end 440. Shaft 434 is slidably received within central lumen 436 and extends proximally of the proximal end of cover 432.

A plurality of runners 442 extend distally from the distal end of shaft 434. Runners 442 line a portion of the inner surface of lumen 436, and slide within the lumen with the shaft. Shaft 434 also has a lumen, in which a core shaft 444 is slidably disposed. Core shaft 444 has a guidewire lumen 446. Guidewire lumen 446 optionally receives an intravascular ultrasound (IVUS) imaging transducer to provide imaging prior to, during, and after deployment of the prosthesis. Nosecone 448 is fixed to the distal end of core shaft 444, and can therefore move independently of runners 442.

Graft 10 is radially compressed and restrained within the plurality of runners 442. In turn, cover 432 prevents runners 442 from expanding outward. Runners 442 are formed from a hard material, and distribute the expansion load of prosthesis 10 over the inner surface of central lumen 436. Advantageously, the prosthesis does not invaginate in the lumen surface, and is thus able to slide relative to the cover in response to a moderate distal force. In the embodiment of Fig. 25, the deploying force is applied proximally against a slider 450 attached to distal end 438 of cover 430, while holding a lure fitting 452 at the distal end of shaft 434. An additional lure adaptor 454 at the distal end of core shaft 444 allows the core shaft to be releasably secured to the shaft 434.

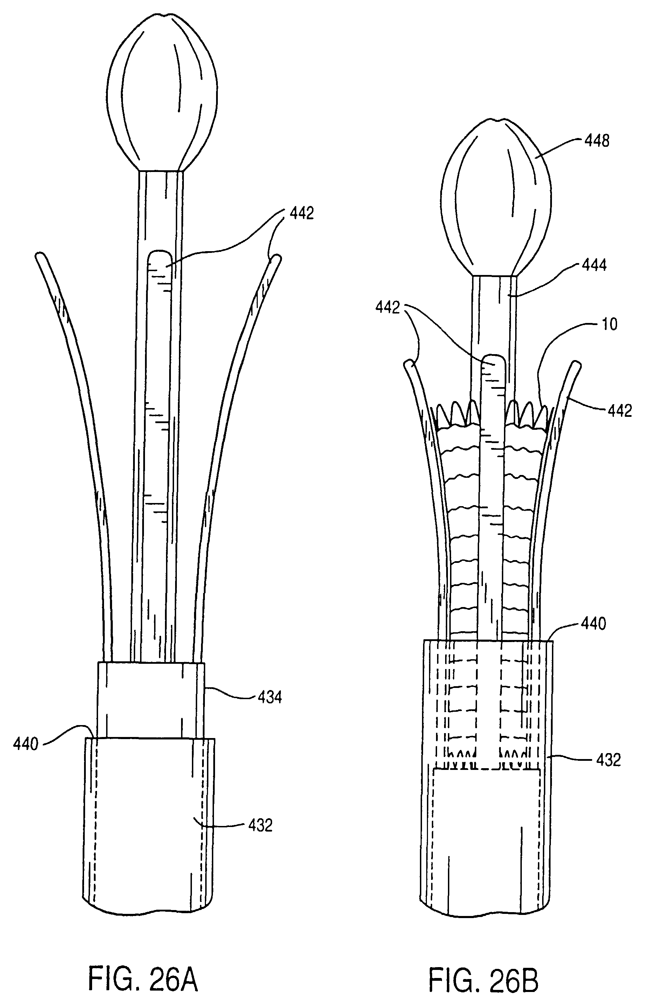

Referring now to Figs. 26A and 26B, loading of graft 10 into the distal end 440 of cover 432 is facilitated by use of runners 442. As seen in Fig. 26A, extending shaft 434 distally allows runners 442 to flex outward. Graft 10 may be inserted between the outward flexed runners and compressed by withdrawing runners 442 and shaft 434 into the distal end 440 of cover 432. Nosecone 448 and core shaft 444 are shown attached to shaft 434 during loading. Alternatively, nosecone 448 may be attached to core shaft 444 after the loading of prosthesis 10. Prosthesis 10 is preferably formed of a heat memory alloy such as Nitinol™. To maintain graft 10 in a compressed state, the loading process may be done in a cold environment, such as that provided by a cold spray, liquid nitrogen, freon, an air vortex, or the like.

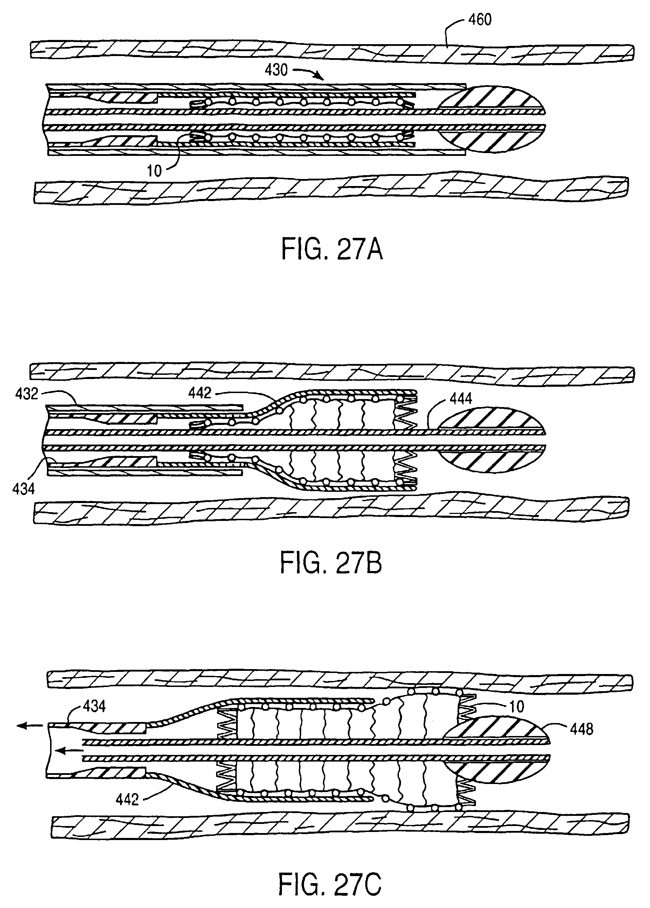

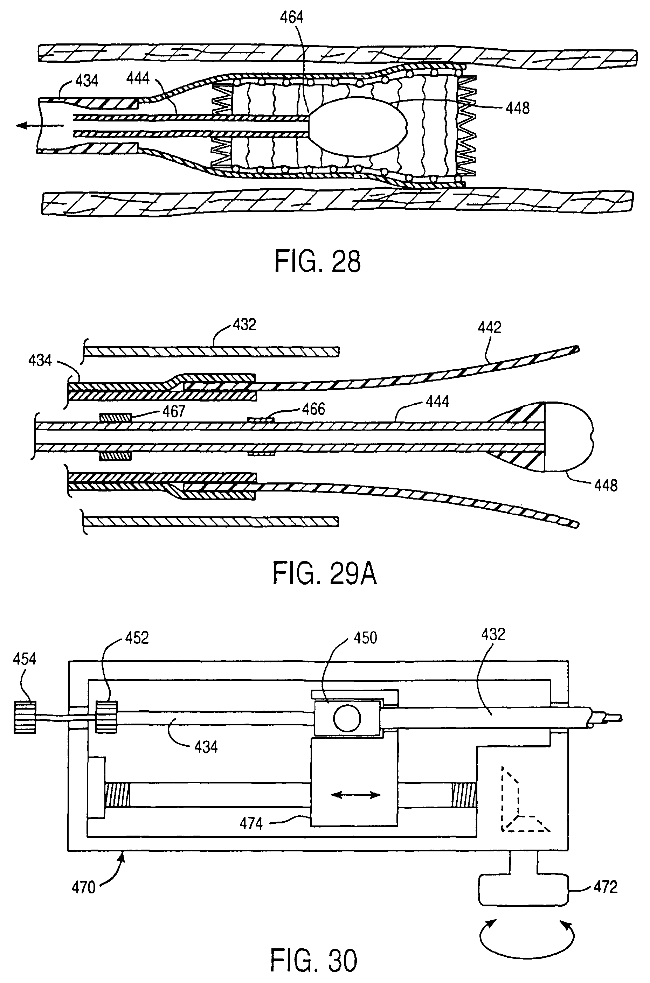

Referring now to Figs. 27A through 28, placement of graft 10 within a body lumen 460 begins by positioning catheter 430 at a target location. As illustrated in Fig. 27B, graft 10 is allowed to expand by retracting cover 432, proximally relative to shaft 434 and core shaft 444. As cover 432 is retracted, runners 442 maintain their axial position, sliding over the inner surface of cover 432. Once the graft 10 has fully expanded within body lumen 60, it is axially anchored by expansion against the lumen wall between the runners. Runners 442 may then be retracted proximally with shaft 434 and nosecone 448. The hard surface of runner 442 allows shaft 434 to be retracted smoothly, with little possibility of dragging graft 10 from the target position. The graft cover may also help to reduce friction during deployment. The possibility of dragging the prosthesis is further reduced by retracting nosecone 448 having a tapered proximal end 464 independently from shaft 434, as illustrated in Fig. 28. Finally, it will be recognized that the runners may also be used to help recapture a partially-deployed prosthesis.

Referring now to Fig. 29A, the elements of the present graft delivery catheter will be described. Cover 432 must be strong enough to withstand the expansion force of graft 10 but must also be flexible to allow intravascular atraumatic maneuvering. Cover 432 is optionally formed of a high strength thermoplastic elastomer such as Hytrel™. Alternatively, cover 432 may be formed of a braided reinforced polymer tubing or a linear reinforced tubing, preferably having fibers of a polyamide such as Kevlar™, Spectra™, or the like, embedded to improve tensile strength without reducing flexibility. Preferably, the cover includes a radiopaque contrast medium, e.g., a B₄SO₄ compound, to allow imaging of the placement of catheter 30 within a body lumen using fluoroscopy. Shaft 434 is preferably formed from PEEK, nylon, or the like, to provide column strength. Runners 442 are formed from a high strength biocompatible alloy such as Nitinol™, stainless steel, or a stainless steel alloy. Runners 442 are bonded to shaft 434, preferably being laminated between inner and outer layers of nylon, a thermoplastic elastomer such as Pebax™, or the like. Core shaft 444 is also preferably formed of PEEK. Nosecone 448 may be formed of stainless steel and bonded to the distal end of core shaft 444, or may alternatively be molded of a radiopaque plastic comprising Pebax™, nylon, Hytrel™, or the like. In any case, nosecone 448 preferably includes a radiopaque element, thereby giving an indication of the location of the distal end of graft 10 during fluoroscopically guided prostheses placement. In certain embodiments, core shaft 444 further supports marker ring 466, comprising platinum, barium, or the like, to provide a sharp radiographic contrast. Optionally, distal force imparting structure 467 is bonded to the core shaft to slide the compressed prosthesis distally over the runners.

Referring now to Fig. 29B, a helical shaft 435 provides high column strength with flexibility. Helical shaft 435 is formed from a tightly wound, high strength metal, preferably comprising stainless steel. Helical shaft 435 is easily welded to runners 442, where similar metals are used for both. Alternatively, runners 442 are laminated to helical shaft 435 with inner and/or outer layers of nylon, Pebax™, or the like. A composite cover 433 comprising polymer reinforced tubing having braided or linear Kevlar™, Spectra™, or the like, further enhances flexibility of the delivery catheter of the present invention.

The delivery catheters of the present invention significantly reduce the force required to deploy a prosthesis within a body lumen. Nonetheless, the force required to withdraw cover 432 remains substantial. For this reason, the present invention further provides a housing 470 to be attached to the distal end of shaft 434, as illustrated in Fig. 30. Rotation of handle 472 moves follower 474 along a linear screw. Slider 450 at the proximal end of cover 432 is driven axially by the movement of the follower. Cover 432 is withdrawn during deployment of the prosthesis by articulating handle 472 so as to drive slider 450 toward lure fitting 452 at the proximal end of shaft 434. The force required to withdraw the cover is typically on the order of 1 to 10 lbs., requiring only a modest mechanical advantage. However, a mechanical advantage ratio in the range from 5 to 50, as measured from the linear travel at the outside edge of handle 472 to the linear motion of follower 474, provides a highly controlled deployment, Clearly, a wide variety of mechanical linkages are available to provide such a mechanical advantage. It is particularly advantageous to provide a mechanism which allows manipulation with a single hand, as this leaves the alternate hand free to manipulate the cover relative to an introducer sheath. It will be noted that housing 470 allows independent manipulation of core shaft 444 using second lure fitting 454, as described above regarding Fig. 28.

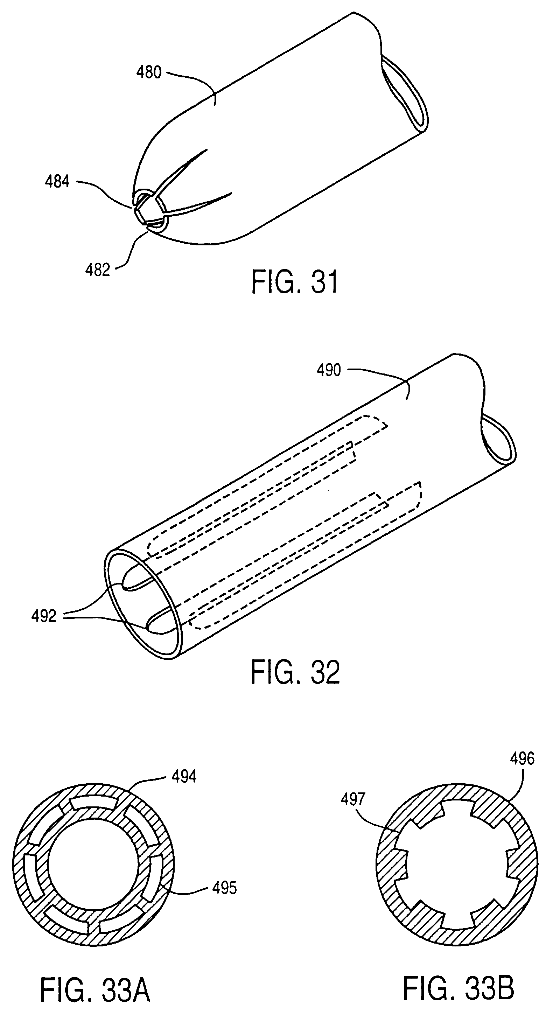

Referring now to Fig. 31, an alternative cover 480 provides an atraumatic distal end 482 with a reduced nosecone diameter, or, alternatively, no nosecone at the distal end of core shaft 444. Atraumatic cover 480 includes a series of splits 484 to allow the distal tip of atraumatic cover 480 to open during deployment of prosthesis 10.

Referring now to Fig. 32, a further alternative cover 490, having runners 492 embedded within the central lumen, will also reduce the friction between the prosthesis and the cover during prosthesis placement. Furthermore, such a structure eliminates any danger of injury to the walls of a body lumen during placement by a distal movement of the exposed runners. Moreover, similar safety advantages could be obtained using the delivery catheter of Fig. 25 by retaining runners 442 within cover 432 during deployment of prosthesis 10. An alternative structure must be provided to apply a distal force against the prosthesis, such as distal force imparting structure 467 shown in Fig. 29A.

Referring now to Figs. 33A and 33B, alternative cross sections 494 and 496 for a delivery catheter tubular cover or shaft will provide additional column strength without a corresponding increase in stiffness. Slots 495 are also suitable for receiving the runners, thus forming the runner/shaft laminated bond. Indents 497 may receive the free distal portion of the runners to prevent rotation of the prosthesis relative to the cover during manipulation of the shaft. Alternatively, a smooth cover lumen facilitates such rotation by allowing the runners to slidingly rotate against the cover lumen surface.

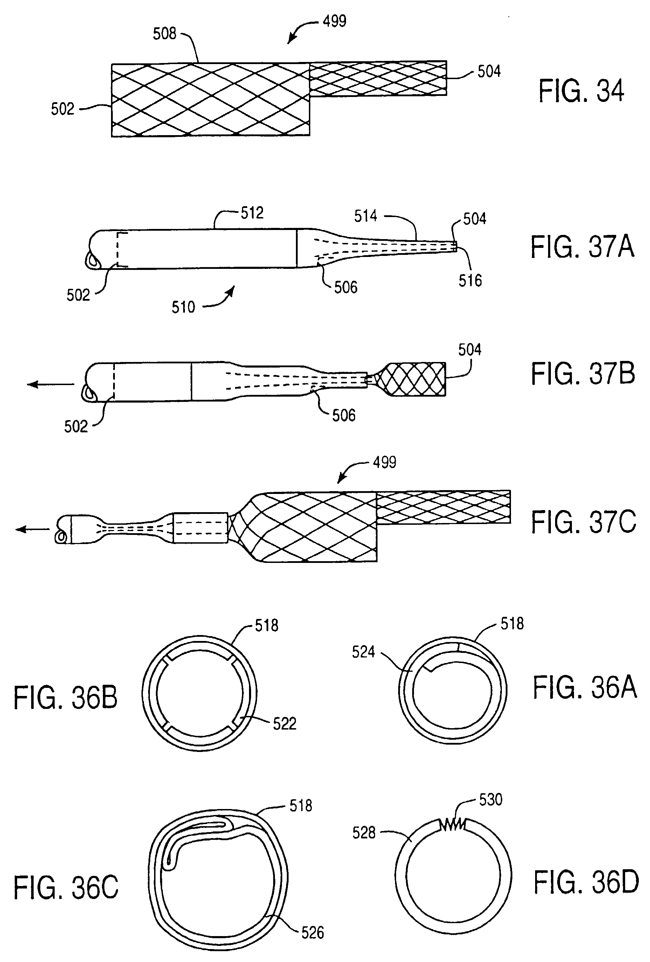

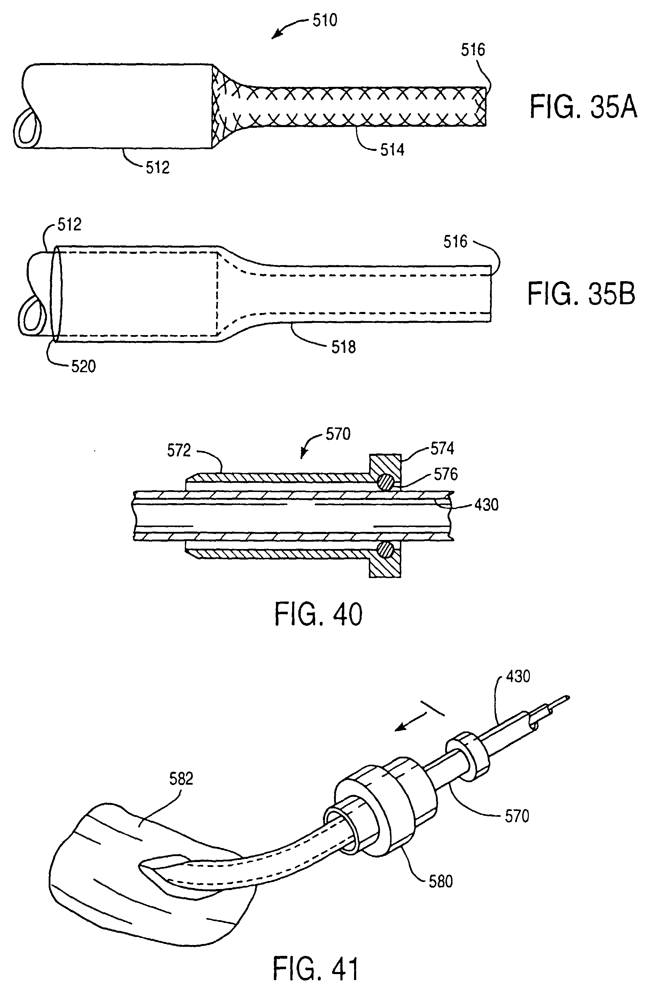

A second exemplary prosthesis structure for use with the delivery catheter of the present invention is illustrated in Fig. 34. Branched prostheses are particularly useful for treatment of aortic aneurysms which extend distally into one or both of the iliac arteries. Branched prosthesis 499 has a large diameter end 502 and a small diameter end 504. Large diameter end 502 features a large common lumen which is in open communication with a first branched lumen of small diameter end 504. The common lumen is further opened at branch 506. Branched prosthesis 499 is formed of a perforate tubular member similar to that used in graft 10. Branched prosthesis 499 may further include a liner as described above.

Treatment of aortic aneurysms using branched prostheses requires placement of the large diameter end in the abdominal aorta with the small diameter end extending into one of the iliac arteries. It will further be understood that it is critical to have branch 506 correctly oriented toward the alternate iliac artery for proper blood flow, whether or not a second tubular prosthesis is placed within open branch 506 and extending into the alternate iliac artery.

Referring now to Figs. 35A and 35B, an exemplary expandable cover 510 for use with branched prostheses comprises a tube 512 and a radially expandable cylindrical structure 514 extending distally of body 512. Body 512 is formed of material similar to that used for cover 432 of the delivery catheter of Fig. 25. Expandable structure 514 comprises a braided, expandable mesh tubing which is bonded or molded into body 512. The mesh tubing expands easily, and is unable to radially compress branched prosthesis 499, but provides a relatively hard surface against which the branched prosthesis can slide distally during deployment. An elastomeric outer coating 518 is disposed over the entire length of the expandable structure and extends proximally onto body 512. Elastomeric outer coating 518 comprises rubber, latex, silicone, polyurethane, a thermoplastic urethane such as C-FLEX™, or the like, and provides the radial compression which retains branched prosthesis 499 in a radially compressed mode.

Referring now to Figs. 36A to 36D, alternative radially expandable cylindrical structures may be formed by extending the material of body 512, and cutting a series of slits to form extended arms 522. Alternatively, a single slit may be used, and the material may be overlapped to form a coil 524. In a still further alternative, a pliable material may be folded over and extended from body 512 to form a folded tube 526. Folded tube 526 is preferably formed of a low friction, pliable material such as PTFE or the like. In yet another alternative structure, open tube 528 having a biasing means 530, such as a metal spring or an elastomeric material, may also be used.

The use of expandable cover 510 will be described with reference to Figs. 37A-37C. Branched prosthesis 499 is loaded into expandable cover 510 with a small diameter end 504 of the prosthesis in a distal expandable structure 514. Large diameter end 502 of branched prosthesis 499 is radially restrained within body 512. The outer diameter of expandable structure 514 is less than the outer diameter of body 512, advantageously allowing the distal end of the delivery catheter to extend into the narrower iliac arteries during a superior placement. Once branched prosthesis 499 is properly positioned at a target location within the delivery catheter, expandable cover 510 may be withdrawn proximally. Expandable structure 514 expands to pass the large diameter end 502 as branch 506 slides distally. Large diameter end 502 and body 512 which restrains it need never enter the restricted diameter of the iliac artery. Similarly, the present expandable cover will be useful for placement of any prosthesis having a distal taper, a smaller distal segment, or a small end and a large portion along its axis.

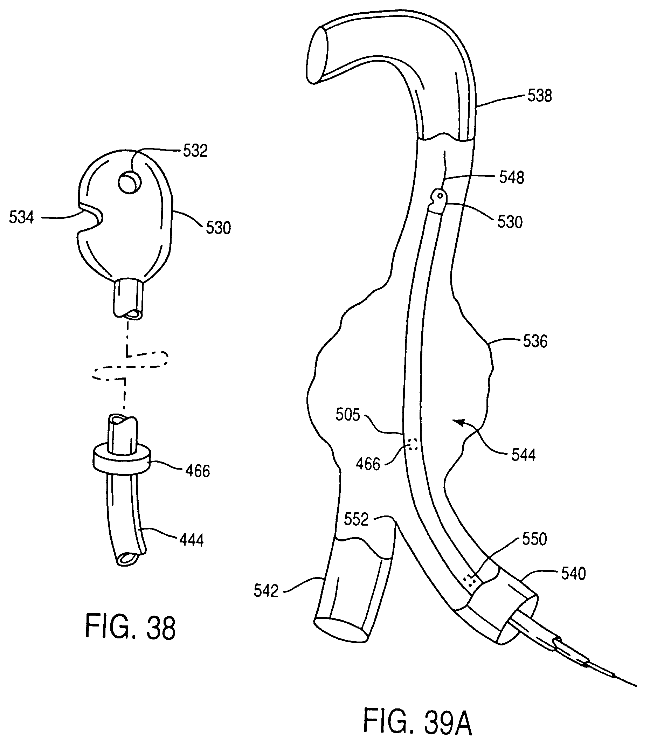

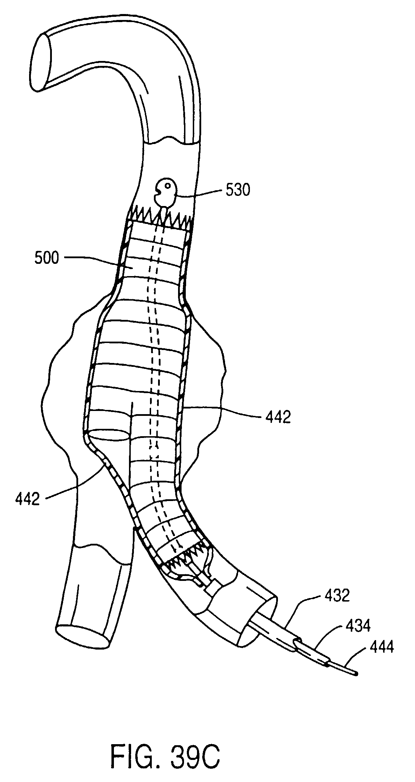

Referring now to Fig. 38, an alternative embodiment of the present nosecone which is particularly useful during the orientation of branched prostheses will be described. An orientation indicating nosecone 530 comprises a stainless steel or other radiopaque structure having a hole 532 and a notch 534. When imaged using fluoroscopy, radiography, or other known imaging modalities, orientation indicating nosecone 530 will provide an indication of the orientation of a branch prior to deployment of a branched prosthesis or stent. For an inferior approach, marker ring 466 is a predetermined distance proximal of a branch 505 when a branched graft 500 is radially restrained within the delivery catheter (see Fig. 39A), thus providing a branch axial marker. A wide variety of asymmetric radiopaque shapes and markers may be used with the present delivery catheter system. It is preferable that the markers remain in position during withdrawal of the cover, and that the markers clearly identify the orientation of a branch 505. It is particularly prefered that marker ring 466 be toward the branch end from branch 505 to ensure the branch is not placed within the iliac artery.

Referring now to Fig. 39A-39E, a method of placement of a branched prosthesis within an aortic aneurysm using the present delivery catheter having the orientation indicating nosecone 530 will be described in regard to branched graft 500. An aneurysm 536 located on an abdominal aorta 538 in close proximity to first and second iliacs 540, 542 is to be treated with a branched graft. A delivery catheter 544 having orientation indicating nosecone 530 is positioned from an inferior placement using a guidewire 548. The axial position and length of branched graft 500 is indicated by orientation indicating nosecone 530 and by a proximal indicator 550, which is structural similar to marker ring 466, and is affixed to the core shaft adjacent to the proximal end of the prosthesis. Marker ring 466 is used as a branch axial indicator, and is placed distally of the body lumen branch 552 to ensure that the branch of branching graft 500 remains within the common lumen. Furthermore, the rotational orientation of the graft is indicated by notch 534 on orientation indicating nosecone 530. The precise rotational orientation may be determined by lining up hole 532 with the imaging energy stream.

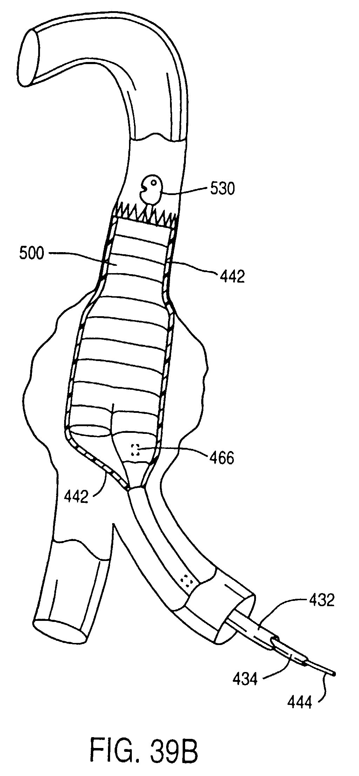

Once delivery catheter 544 is properly positioned, the cover 432 may be withdrawn proximally as illustrated in Fig. 39B. Shaft 434 and core shaft 444 remain at the target position, allowing runners 442 to slide over the central lumen 436 of cover 432. Additionally, orientation indicating nosecone 530 and marker ring 466 remain at the axial location of branched graft 500. Alternatively, the cover 432 may be withdrawn a little at a time, with the runners withdrawn back simultaneously into the cover each time. In a still further alternative, runners 442 may remain within cover 432, and branched graft 500 may be held at the target position by a force imparting structure attached to core shaft 444. Such force imparting structures are more fully explained in application Serial No. 08/294,021, previously incorporated herein by reference. Optionally, core shaft 444 remains stationary while withdrawing the runners so that nosecone 530 does not drag branched prosthesis 500 from the target location.

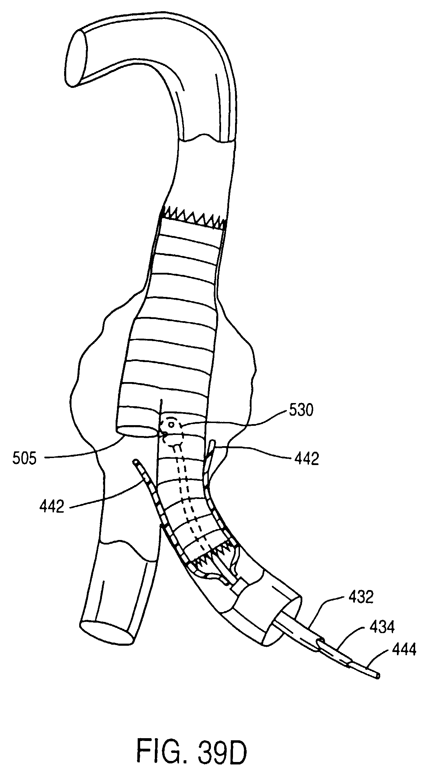

Referring now to Fig. 39D, once cover 430 has been withdrawn, runners 442 and nosecone 530 are withdrawn simultaneously through the branched prosthesis. Core shaft 444 and shaft 434, which are optionally attached, are withdrawn proximally. The runners may be withdrawn into cover 432, or the delivery catheter may move proximally with the runners remaining exposed. Alternatively, core shaft 444 is withdrawn independently of shaft 434 in a proximal direction. Very little force should be required to withdraw nosecone 530 independently of runners 442. Any friction which is encountered indicates that nosecone 530 may be snagged, for example, on branch 505. The surgeon may then manipulate core shaft 444 free nosecone 530, and thereby overcome such a snag. Once orientation indicating nosecone 530 is fully withdrawn shaft 434 and runners 442 may be retracted, leaving branched graft 500 in place.

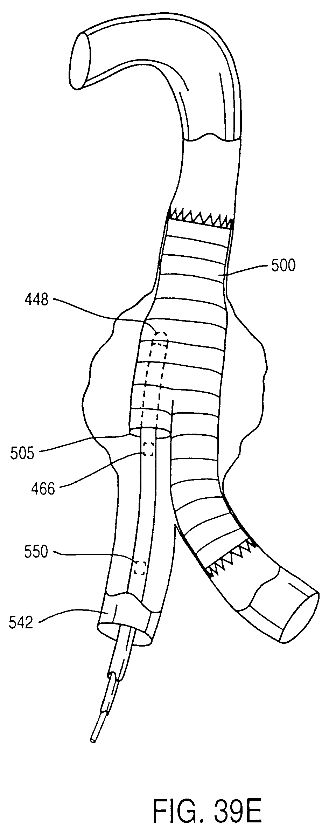

Referring now to Fig. 39E, a secondary tubular prosthesis may be placed extending from branch 505 of branched prosthesis 500 down to second iliac 542. Delivery catheter 430 has a standard nosecone 448, as the rotational orientation is not critical. The distal and proximal ends of the prosthesis may be indicated by the nosecone 448 and proximal marker 550. It is preferable that marker ring 466 be located a predetermined distance proximal of the distal end of the prosthesis. Delivery catheter 30 is then axially positioned with marker ring 466 proximal of branch 505 to avoid the distal end of the prosthesis extending too far into branched graft 500 and folding over in the flow. Marker ring 466 thus acts as a safety marker.

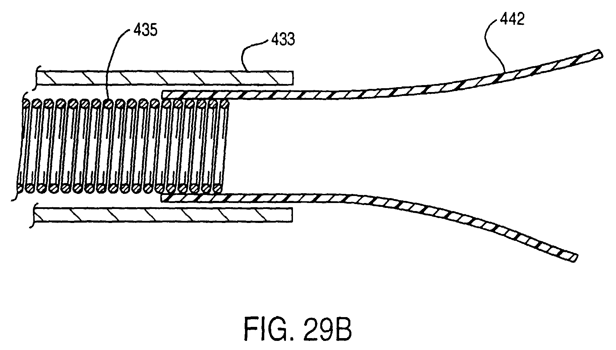

The apparatus and methods of the present invention provide a cover which is smoothly retractable relative to a radially compressed prosthesis. However, a substantial amount of friction may be encountered at the introduction sheath where the present catheters enter a patient's body. Introduction sheaths must provide hemostasis for catheters and other invasive surgery apparatus of various sizes and configurations. Such introduction sheaths typically include a resilient sealing valve which radially compresses the outermost layer of the catheter. Such introduction sheath valves impose a substantial amount of friction against the catheter, making smooth withdrawal of a delivery catheter cover problematic. Therefore, the present invention further provides a friction reducer tube, as illustrated in Fig. 40.

Friction reducer tube 570 comprises a distally tapered tube 572, a proximal o-ring housing 574, and an o-ring 576. Tapered tube 572 is slightly larger in diameter than cover 430 of the present delivery catheter. O-ring 576 provides hemostasis against the cover, but slides axially with little friction.

Referring now to Fig. 41, an introduction sheath 580 is inserted into the patient body 582. Delivery catheter 430 is slidably disposed within friction reducer tube 570, and is introduced into the patient body through introduction sheath 580. Friction reducer tube 570 may then be slid distally so that the hemostasis valve rides over tapered tube 572. To deploy a tubular prosthesis, one surgeon retracts cover 430, while another surgeon manipulates the distal end of cover 432 relative to shaft 434, ideally using a mechanical advantage mechanism as illustrated in Fig. 30.

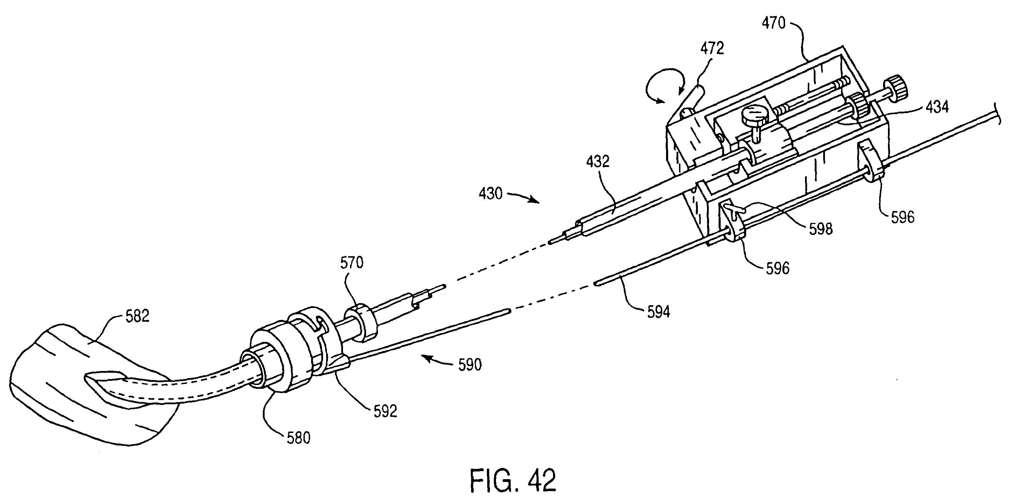

Referring now to Fig. 42, a brace 590 optionally restrains the prosthesis at the target location while withdrawing cover 432. Brace 590 attaches to introducer sheath 580 with a locking collar 592. Bar 594 extends proximally from locking collar 592, and is slidably received by tabs 596 protruding from housing 470. Once the prosthesis is positioned at the target location, a set screw 598 is tightened to fix the distance between the proximal end of delivery catheter 430 and the introduction sheath 580. Rotating handle 472 thus withdraws cover 430 proximally through introduction sheath 580 without distally advancing shaft 434. This minimizes the danger of advancing the exposed runners into the lumen wall during deployment, and thus allows deployment by a single surgeon. The compressive load on bar 594 is reduced by friction reducer tube 570.

A wide variety of compression bearing structures could be used in place of bar 590. A telescoping tube with single or multiple overlapping sections having set screws would eliminate the protruding proximal end of the rod. Such a telescoping tube may optionally surround catheter 430 between the introducer sheath and housing. Alternatively, a flexible tube having good column stiffness disposed over the delivery catheter also prevents axial movement of the prosthesis, and avoids the long, rigid, and potentially cumbersome bar structure. Such a flexible tube preferably comprises a tightly wound coil analogous to flexible shaft 435 shown in Fig. 29B. Although a fixed length tube may be used, telescoping flexible overlapped tubes, usually having a locking device such as set screws, compressive clamps, or the like, are preferred.

The brace of the present invention may advantageously be used with alternative proximal housings having a wide variety of mechanisms for translating the cover relative to the shaft, including electric motors, foot operated linkages, and the like.

Although the foregoing invention has been described in some detail by way of illustration and example, for purposes of clarity and understanding, certain changes and modifications will be obvious to those of skill in the art. For example, the present cover and/or runners may be attached to the elongate shaft as a cartridge, preferably preloaded with a prosthesis. Thus, the scope of the present invention is limited only by the appended claims.

Thus the invention concerns a delivery catheter for positioning radially compressible prostheses, said catheter comprising:
an elongate flexible shaft having a proximal end and a distal end;
a retaining structure near the distal end of the shaft which releasably holds the axial position of the prosthesis on the shaft; and
a sheath slidably received over the shaft to cover the prosthesis while said prosthesis is axially held on the shaft by the retaining structure.

The following embodiments may be provided:

The sheath may have a fixed or deployable outwardly flared distal end to facilitate proximal and distal motion over the prosthesis, in which case means may be provided on the sheath for reconfiguring the distal end of the sheath between a non-flared configuration and a flared configuration, wherein the reconfiguring means may comprise an inflatable bladder and means for inflating said bladder, wherein the bladder flares the distal tip of the sheath outward when inflected.

The distal end of the catheter may comprise a resilient structure having a fixed flared configuration.

The delivery catheter may comprise an axially translatable cap structure which in a proximal position captures the distal end to hold it in a non-flared configuration.

The delivery catheter may comprise a containment sleeve slidably disposed over the sheath, wherein the sleeve closes the flared end when distally advanced and allows the flared end to open when proximally retracted.

The flared distal end of the sheath may comprise a structure including heat memory alloy components which define the flared structure of the distal end of the sheath at body temperatures and which assume a non-flared configuration at below body temperature.

The delivery catheter may comprise a tubular membrane attached at one end of the shaft at a location proximal of the retaining structure and at the other end to the inside of the sheath, wherein the membrane envelops the prosthesis when said prosthesis is radially compressed within the sheath and wherein the membrane everts as the sheath is retracted proximally relative to the shaft.

The retaining structure of the delivery catheter may comprise at least one locking stay on the shaft, in which case the locking stay may be disposed over the outside of the shaft and extends radially outward by a distance sufficient to engage the interior surface of the sheath, whereby the stay will extend through an aperture in the prosthesis to hold the prosthesis axially in place relative to the shaft as the sheath is axially translated relative to the shaft.

The retaining structure may comprise at least two circumferentially spaced-apart locking stays disposed over the shaft.

The retaining structure may comprise a cover which is detachably secured over the radially compressible prosthesis and means operable from the proximal end of the flexible shaft for detaching the cover from over the prosthesis to release said prosthesis, in which case the cover may be a cylinder and the detaching means is a mechanism selected from the group consisting of (a) a cord for splitting the cylinder along at least one axial or spiral line by drawing proximally on the cord, (b) an axially translatable end cap which captures a plurality of resilient, radially outwardly flared axial elements of the cylinder, and (c) means for axially separating two halves of the cylindrical cover.

The delivery catheter may comprise a journal sleeve slidably disposed over the sheath, wherein the journal sleeve permits external anchoring of the catheter while the sheath can still be translated relative to the shaft. The delivery catheter may comprise a lock which selectively fixes the shaft relative to the journal sleeve while permitting the sheath to be axially translated relative to the shaft and journal sleeve.

The delivery catheter may be provided with a shaft having a coupling mechanism spaced inward for the distal end and the sheath has a coupling mechanism spaced inward from the distal end, whereby different distal ends of the catheter may be attached.

The invention further concerns a catheter system comprising a radially compressible prosthesis held by the retaining structure of the shaft of the delivery catheter as defined before.

The prosthesis may be axially anchored by the retaining structure and may be radially compressed by the sheath itself. It is further possible that the prostheses may be axially anchored and radially compressed by the retaining structure.

Additionally the invention concerns a delivery catheter for use with a prosthesis cartridge including a shaft extension, a sheath extension slidably received over the shaft extension, and a prosthesis radially compressed over the shaft extension and within the sheath extension, said delivery catheter comprising:
an elongate flexible shaft having a proximal end, a distal end, and a coupling member at the distal end which couples to a proximal end of the shaft extension of the prosthesis cartridge; and
an elongate member slidably attached to the shaft and having a proximal end, a distal end, and a coupling member at the distal end which couples to a proximal end of the sheath extension of the prosthesis cartridge;
and a prosthesis cartridge for use with a delivery catheter including an elongate flexible shaft and an elongate member slidably attached to the shaft, said prosthesis cartridge comprising:
a shaft extension having a proximal end, a distal end, and a coupling member at the proximal end which couples to a distal end of the elongate flexible shaft of the delivery catheter;
a sheath extension having a proximal end, a distal end, and a coupling member at the proximal end which couples to a distal end of the elongate member of the delivery catheter; and
a prosthesis radially compressed over the shaft extension and within the sheath extension.

The invention further concerns a set of prosthesis cartridges comprising a plurality of prosthesis cartridges as defined in the last paragraph, wherein at least some of said cartridges comprise prostheses having different lengths, and/or wherein the cartridge is contained in a sterile package.

A method for preparing a prosthesis delivery catheter comprises:
providing a delivery catheter including:
   (a) an elongate flexible shaft having a proximal end, a distal end, and a coupling member at the distal end; and
   (b) an elongate member slidably attached to the shaft and having a proximal end, a distal end, and a coupling member at the distal end;
providing a prosthesis cartridge including:
   (a) a shaft extension having a proximal end, a distal end, and a coupling member at the proximal end;
   (b) a sheath extension having a proximal end, a distal end and a coupling member at the proximal end; and
   (c) a prosthesis radially compressed over the shaft extension and within the sheath extension; and
attaching the coupling member on the shaft extension to the coupling member on the flexible elongate shaft and attaching the coupling member on the sheath extension to the coupling member on the elongate member.

Furthermore, the invention concerns a delivery catheter for placement of a radially compressible prosthesis, the catheter comprising:
an elongate flexible shaft structure having a proximal end, a distal end, and a prosthesis receptacle near the distal end;
at least one runner disposed over the prosthesis receptacle of the shaft structure; and
a tubular cover slidably disposed over at least the prosthesis receptacle of the shaft;
   wherein the cover restrains the prosthesis against the at least one runner.

The following embodiments may be provided:

The delivery catheter may further comprise a plurality of axially disposed elongate runners affixed together at their proximal ends;
the proximal ends of the runners may be proximal of the prosthesis receptacle, and the runners may extend distally of the constrained prosthesis;
the runners may be flexible and affixed to the shaft structure so that the runners remain over the prosthesis receptacle as the cover is axially translated over the shaft structure;
the runners and the shaft structure may axially restrain the prosthesis as the cover is proximally translated from the prosthesis receptacle;
the shaft structure may include a core shaft which is axially slidable relative to the runners, the core shaft having a distal force imparting structure which axially restrains the prosthesis as the cover and the runners are axially translated from the prosthesis receptacle;
the runners may be affixed to the cover, and the shaft structure may axially restrain the prosthesis as the runners and the cover are proximally translated from the prosthesis receptacle;
the runners may comprise strips of material selected from the group consisting of shape memory alloy, stainless steel, and stainless alloy, titanium, and titanium alloy;
the delivery catheter may further comprise an asymmetric rotation marker adjacent to the prosthesis receptacle, the rotation marker being radiopaque;
the delivery catheter may further comprise a housing at the proximal end of the shaft structure, the housing including a handle and mechanical advantage mechanism which multiplies a force against the handle and applies a multiplied force axially against the cover.

Additionally, the invention concerns a delivery catheter for placement of a compressible prosthesis, the catheter comprising:
an elongate flexible shaft structure having a proximal end, a distal end, and a prosthesis receptacle near the distal end;
a plurality of elongate runners extending distally from the distal end of the shaft structure, the runners having a hardness greater than that of the cover; and
a tubular cover slidably disposed over the prosthesis receptacle on the shaft;
   wherein the runners are radially constrained toward the prosthesis receptacle when the cover is distally extended over the prosthesis receptacle and are radially released when the cover is proximally retracted.

The following embodiments may be provided:

The delivery catheter may have a shaft structure which comprises a core shaft slidably disposed within the runners, the core shaft having a guidewire lumen and means for releasably attaching the core shaft to the runners at the proximal end of the shaft structure, wherein the core shaft and distal end of the cover are capable of presenting an atraumatic distal catheter end;
the core shaft may include a proximally tapered nosecone at the distal end of the shaft structure;
the shaft structure may comprise a core shaft attached to and extending distally from the proximal end of the runners, the core shaft having a guidewire lumen, wherein the core shaft and distal end of the cover are capable of presenting an atraumatic distal catheter end;
the runners may comprise strips of material selected from the group consisting of shape memory alloy, stainless steel, stainless alloy, titanium, and titanium alloy;
the runners may be affixed to the shaft between inner and outer layers of a material selected from the group consisting of thermoplastic elastomer, Nylon, and stainless steel;
the plurality of runners may comprise between 1 and 20 runners, and wherein each runner comprises a strip which is longer than the prosthesis receptacle, a width in the range between .010 and .090 inch, and a thickness in the range between .001 and .020 inch;
the cover may comprise a material selected from the group consisting of thermoplastic elastomer, braided reinforced polymer, and polyamide fiber reinforced polymer;
the delivery catheter may further comprise an asymmetric rotation marker adjacent to the prosthesis receptacle, the rotation marker being radiopaque;
the delivery catheter may further comprise a housing at the proximal end of the shaft, the housing including a handle and a mechanical advantage mechanism for multiplying a force against the handle and applying a multiplied force proximally against the cover;
the mechanical advantage mechanism may provide a mechanical advantage in the range from 5 to 50;
the delivery catheter may further comprise a friction reducer tube slidably disposed about the cover and insertable within a valve seal of an introduction sheath, the friction reducer tube including sealing means against the cover.

Further, the invention concerns a delivery catheter for placement of a resilient, radially compressible prosthesis within a patient body, the catheter passing through an introduction sheath having a valve seal, the catheter comprising:
an elongate flexible shaft having a proximal end and a distal end and a lumen therebetween;
a plurality of runners affixed to the shaft at their proximal ends and oriented distally, the runners comprising a high strength metal, the runners being longer than the length of the prosthesis;
a tubular cover slidably disposed over the shaft and runners;
a friction reducer tube slidably disposed over the cover and insertable within the valve seal of the introduction sheath, the friction reducer tube including sealing means against the cover; and
a core shaft slidably disposed within the lumen of the shaft and having means for releasably attaching to the shaft at the proximal end, the core shaft having a guidewire lumen and a nosecone.

The invention concerns also an improved delivery catheter for placement of a branching, radially compressible prosthesis in a branching body lumen, the catheter of the type having an elongate shaft structure including a proximal end, a distal end, an axis therebetween, and a prosthesis receptacle near the distal end, the improvement comprising:
a rotation marker on the shaft structure adjacent to the prosthesis receptacle, the rotation marker having an asymmetric radiographic image, the image being asymmetric about the axis of the shaft structure.

The delivery catheter may further comprise a branch axial marker on the shaft structure within the prosthesis receptacle, and/or
a proximal marker disposed a predetermined distance from the proximal end of the prosthesis receptacle and a distal marker disposed a predetermined distance from the distal end of the prosthesis receptacle, wherein the proximal and distal markers are radiopaque and are movable with the shaft, and/or
the rotation marker may include a radiopaque body having a radiographically clear passage and a radiographically clear notch.

The invention concerns also an improved prosthesis delivery catheter for placement of a secondary radially compressible prosthesis within a branch of a branching prosthesis, the branching prosthesis placed in a branching body lumen, the catheter of the type having an elongate shaft structure including a proximal end, a distal end, and a prosthesis receptacle near the distal end, the improvement comprising:
a radiopaque safety marker on the shaft structure along the prosthesis receptacle, the safety marker a maximum insertion distance from an end of the prosthesis receptacle, the maximum insertion distance corresponding to a maximum distance the secondary prosthesis can safely extend into the branch of the branching prosthesis;
as well as an expandable tip delivery catheter for placement of a radially compressible prosthesis having a large diameter portion and a small diameter end, the catheter comprising:
an elongate flexible shaft having a proximal end, a distal end, and a prosthesis receptacle near the distal end; and
a cover slidably disposed about the shaft, the cover comprising:
a body portion; and
a resilient structure extending distally from the body portion, the resilient structure having an inherent cross-section which is smaller than the body portion;
   wherein the resilient structure expands over the prosthesis receptacle as the cover is withdrawn proximally over the shaft,
   wherein the resilient structure may comprise a braided expandable mesh tubing and an elastomeric material disposed over the mesh tubing.

## Claims

1. A delivery catheter for positioning radially compressible prostheses, said catheter comprising:
an elongate flexible shaft having a proximal end and a distal end;
a retaining structure near the distal end of the shaft which releasably holds the axial position of the prosthesis on the shaft; and
a sheath slidably received over the shaft to cover the prosthesis while said prosthesis is axially held on the shaft by the retaining structure.

2. The delivery catheter of claim 1, wherein the sheath has a fixed or deployable outwardly flared distal end to facilitate proximal and distal motion over the prosthesis.

3. The delivery catheter of claim 1 or 2, further comprising means on the sheath for reconfiguring the distal end of the sheath between a non-flared configuration and a flared configuration.

4. The delivery catheter of anyone of the claims 1 to 3, wherein the reconfiguring means comprises an inflatable bladder and means for inflating said bladder, wherein the bladder flares the distal tip of the sheath outward when inflected.

5. The delivery catheter of anyone of the claims 2 to 4, wherein the distal end of the catheter comprises a resilient structure having a fixed flared configuration.

6. The delivery catheter of claim 5, further comprising an axially translatable cap structure which in a proximal position captures the distal end to hold it in a non-flared configuration.

7. The delivery catheter of claim 5, further comprising a containment sleeve slidably disposed over the sheath, wherein the sleeve closes the flared end when distally advanced and allows the flared end to open when proximally retracted.

8. The delivery catheter of anyone of the claims 2 to 7, the flared distal end of the sheath comprises a structure including shape memory alloy components which define the flared structure of the distal end of the sheath at body temperatures and which assume a non-flared configuration at below body temperature.

9. The delivery catheter of anyone of the claims 2 to 8, wherein the flared distal end of the sheath comprises a structure including a nickel titanium alloy.

10. The delivery catheter of anyone of the claims 1 to 9, further comprising a tubular membrane attached at one end of the shaft at a location proximal of the retaining structure and at the other end to the inside of the sheath, wherein the membrane envelops the prosthesis when said prosthesis is radially compressed within the sheath and wherein the membrane everts as the sheath is retracted proximally relative to the shaft.

11. The delivery catheter of anyone of the claims 1 to 10, further comprising a journal sleeve slidably disposed over the sheath, wherein the journal sleeve permits external anchoring of the catheter while the sheath can still be translated relative to the shaft.

12. The delivery catheter of claim 11, further comprising a lock which selectively fixes the shaft relative to the journal sleeve while permitting the sheath to be axially translated relative to the shaft and journal sleeve.
